# EUROPÄISCHE PATENTSCHRIFT

(11) **EP 2 599 505 B1**
(45) Veröffentlichungstag und Bekanntmachung des Hinweises auf die Patenterteilung: **14.05.2014**
(21) Anmeldenummer: 11191283.8
(22) Anmeldetag: 30.11.2011
(51) Int. Cl.: A61L 2/08, A61B 5/1486, A61B 5/145

(54) **Verfahren und Sterilisationsvorrichtung zum Sterilisieren eines implantierbaren Sensors**
Method and sterilisation device for sterilising an implantable sensor
Procédé et dispositif de stérilisation pour la stérilisation d'un capteur implantable

(43) Veröffentlichungstag der Anmeldung: 05.06.2013
(73) Patentinhaber: Roche Diagnostics GmbH, 68305 Mannheim (DE); F. Hoffmann-La Roche AG, 4070 Basel (CH); Carl Freudenberg KG, 69469 Weinheim (DE)
(72) Erfinder: Harttig, Herbert, 67434 Neustadt (DE); Heinrich, Ralf, 67365 Schwegenheim (DE); König, Stefan, 64653 Lorsch (DE)
(74) Vertreter: Stößel, Matthias

(56) Entgegenhaltungen:
- DE-A1-102004 033 219
- US-A1- 2009 257 911
- US-A1- 2011 152 644
- US-B1- 6 594 156

## Beschreibung

### Gebiet der Erfindung

Die Erfindung betrifft ein Verfahren zum Sterilisieren eines implantierbaren Sensors sowie eine Sterilisationsvorrichtung zum Sterilisieren eines implantierbaren Sensors. Der Sensor dient der Erfassung mindestens eines Analyten in einem Körpergewebe. Insbesondere kann es sich bei dem Sensor um einen elektrochemischen Sensor handeln, welcher eingerichtet ist, um auf elektrochemischem Wege einen oder mehrere Analyte in einem Körpergewebe qualitativ und/oder quantitativ zu erfassen. Derartige Sensoren werden beispielsweise in der Überwachung von Blutglucosekonzentrationen eingesetzt. Auch andere Einsatzgebiete sind denkbar.

### Stand der Technik

Aus dem Stand der Technik ist eine Vielzahl von Sensoren bekannt, welche vollständig oder teilweise in ein Körpergewebe implantierbar sind und welche der Überwachung bestimmter Körperfunktionen, insbesondere der Überwachung einer oder mehrere Konzentrationen bestimmter Analyten dienen. Ohne Beschränkung weiterer möglicher Ausgestaltungen wird die Erfindung im Folgenden unter Bezugnahme auf eine Blutglucoseüberwachungsvorrichtung beschrieben. Grundsätzlich ist die Erfindung jedoch auch auf andere Arten von Analyten übertragbar.

Neben so genannten Punktmessungen, bei welchen einem Benutzer gezielt eine Probe einer Körperflüssigkeit entnommen und auf die Analytkonzentration untersucht wird, etablieren sich zunehmend auch kontinuierliche Messungen. So etabliert sich beispielweise eine kontinuierliche Glucosemessung im Interstitium, welche auch als Continuous Monitoring, CM, bezeichnet wird, in jüngerer Vergangenheit als wichtige Methode zum Management, zur Überwachung und zur Steuerung beispielsweise eines Diabetes-Status. Mittlerweile kommen dabei in der Regel direkt implantierte elektrochemische Sensoren zum Einsatz, welche häufig auch als nadelartige Sensoren (Needle Type Sensors, NTS) bezeichnet werden. Dabei wird der aktive Sensorbereich direkt an den Messort gebracht, welcher in der Regel im interstitiellen Gewebe angeordnet ist, und beispielsweise unter Verwendung eines Enzyms, beispielsweise Glucoseoxidase, Glucose in elektrischen Strom umgesetzt, der im Verhältnis zur Glucosekonzentration steht und als Messgröße verwendet werden kann. Beispiele derartige transkutaner Messsysteme sind in US 6,360,888 B1 oder in US 2008/0242962 A1 beschrieben.

Aktuelle Continuous Monitoring Systeme sind somit in der Regel transkutane Systeme. Dies bedeutet, dass der eigentliche Sensorteil des Sensors mit den Elektroden, unterhalb der Haut des Benutzers in einem Körpergewebe angeordnet ist. Ein Elektronikteil, welcher oft auch als Auswerte- und/oder Steuerteil oder auch als Patch bezeichnet wird, befindet sich jedoch in der Regel außerhalb des Körpers des Benutzers, also außerhalb des menschlichen oder tierischen Körpers. Der Sensorteil wird dabei in der Regel mittels einer Insertionshilfe appliziert, welche exemplarisch ebenfalls in US 6,360,888 B1 beschrieben wird. Auch andere Arten von Insertionshilfen sind bekannt. Die Tragedauer eines Sensors beträgt in der Regel ca. 1 Woche.

Die vollständige oder teilweise Insertion des Sensors in ein Körpergewebe bedingt in der Regel, dass voll- oder teilweise implantierbare Komponenten des Sensors für den Einsatz am Menschen und/oder Tier nach geltenden Normen zu sterilisieren sind. Bei elektrochemischen Glucosesensoren auf enzymatischer Basis steht das Enzym in der Elektrode eingebettet direkt oder über eine Schutzschicht mit dem Interstitium in Kontakt, d.h. die Elektroden liegen offen. Eine chemische oder thermische Sterilisation scheidet dementsprechend in der Regel aus, da bei derartigen Sterilisationen das Enzym der Elektroden geschädigt würde. Daher kann in aller Regel nur eine Strahlensterilisation eingesetzt werden.

Hierbei stellt sich jedoch das Problem, dass elektronische Komponenten des Sensors in aller Regel einer direkten Bestrahlung, beispielsweise einer Bestrahlung mit Betastrahlung oder Elektronenstrahlung, bei den typischerweise benötigten Strahlendosen (üblicherweise 25 kGy) nicht standhalten. Insbesondere aktive elektronische Komponenten auf Halbleiterbasis, wie beispielsweise hochohmige Verstärkerbauteile oder Potentiostaten, sind üblicherweise nicht in der Lage, Strahlensterilisationen mit Betastrahlung bei den genannten Strahlendosen ohne Funktionseinbuße zu überstehen. Aus dem Stand der Technik sind eine Vielzahl von Verfahren bekannt, mittels derer ein Schutz von Sensorelementen während einer Strahlensterilisation erfolgen kann. So wird beispielsweise in WO 2006/005503 A1 ein Verfahren zur Herstellung integrierter, diagnostischer Testelemente beschrieben. Die Testelemente weisen einen Stechbereich und einen Nachweisbereich auf. Dabei wird der Nachweisbereich auf dem Testelement gegen eine für die Sterilisation verwendete Elektronenstrahlung abgeschirmt. Unter anderem wird auch beschrieben, dass die Testelemente in einer Verpackung angeordnet sein können, und dass die Verpackung so gestaltet sein kann, dass der Nachweisbereich der Testelemente gegen Elektronenstrahlung abgeschirmt wird und eine Bestrahlung der gesamten Verpackung erfolgt.

In US 5,496,302 wird ein Verfahren zur Sterilisierung eines ausgewählten Bereichs eines Produktes und zur Herstellung eines sterilen Produktes aus zwei oder mehr Komponenten, die nicht auf dieselbe Art sterilisiert werden können, beschrieben. Dabei wird ein Schlauchsystem mit einer sterilen Flüssigkeit verwendet, bei dem ein Teil eines Gehäuses mit Elektronenstrahlung sterilisiert wird, während ein anderer Teil durch eine Abschirmung vor den Einwirkungen der Elektronenstrahlen geschützt wird.

In US 2008/0255440 A1 wird eine Sensorverpackung mit einem implantierbaren Sensor beschrieben. Der implantierbare Sensor weist einen Elektrodenbereich und einen elektrischen Kontaktbereich auf. Der Sensor wird durch Betastrahlen sterilisiert, wobei die Verpackung derart ausgestaltet ist, dass der elektrische Kontaktbereich sterilisiert wird, wohingegen der Elektrodenbereich geschützt bleibt. Die Verpackung kann beispielsweise einen Sensorteil vor einem Einfluss weiterer, zur Sterilisierung von Elektronikkomponenten verwendeter Gase schützen.

In US 6,594,156 B1 wird eine Vorrichtung zum Schutz von elektrischen Schaltkreisen während einer hochenergetischen Strahlensterilisation beschrieben. Die Vorrichtung umfasst ein Trägersubstrat und ein schützendes Gehäuse für elektronische Komponenten. Das schützende Gehäuse wird hermetisch an das Trägersubstrat der elektronischen Komponenten gekoppelt und schützt diese vor den Auswirkungen der Strahlensterilisation. Weiterhin werden elektronische Schaltkreise beschrieben, die mit einer vorbestimmten Strahlendosis sterilisiert sind und bei denen nach der Sterilisation der Verstärkungsfaktor nicht über ein bestimmtes Maß hinaus reduziert ist und die Proportionalität zwischen Kollektorstrom und Basisstrom erhalten bleibt.

Die aus dem Stand der Technik bekannten Lösungen weisen eine Mehrzahl technischer Herausforderungen oder sogar Nachteile auf. So sind die bekannten Lösungen in der Regel nicht darauf eingerichtet, einen Sensor oder ein Sensorsystem zu sterilisieren, welche ohne weitere komplexe Schritte an einem menschlichen Körper angewandt werden können. Insbesondere wird in der Regel nicht berücksichtigt, dass ein solches Sensorsystem die Haut berührende Teile aufweist, die einerseits steril sein müssen, andererseits jedoch mit der empfindlichen Elektronik in direktem Kontakt stehen. Damit das Sensorsystem im komplett notwenigen Umfang sterilisiert ist, müssen bei bekannten Vorrichtungen und Verfahren oftmals mehrere, aufeinanderfolgende Sterilisationsverfahren eingesetzt werden, wie beispielsweise eine Strahlensterilisation und eine chemische Sterilisation.

Weiterhin sind die aus dem Stand der Technik bekannten Vorrichtungen häufig derart komplex ausgestaltet, dass diese in großtechnischen Prozessen nahezu nicht eingesetzt werden können. So ist in der Regel vor und nach der Strahlensterilisation eine aufwendige Vorbereitung, bzw. Nachbereitung der Sterilisationsvorrichtung erforderlich, während derer eine Nachverkeimung der Sensoren erfolgen kann. Gleichzeitig sind die Sterilisationsvorrichtungen jedoch derart aufwendig, dass diese in der Regel nicht als ganze, mitsamt der Abschirmvorrichtung, an einen Endkunden ausgeliefert werden könnten.

### Aufgabe der Erfindung

Es ist daher eine Aufgabe der vorliegenden Erfindung, ein Verfahren und eine Sterilisationsvorrichtung zum Sterilisieren eines implantierbaren Sensors zur Erfassung mindestens eines Analyten in einem Körpergewebe bereitzustellen, welche die Nachteile der oben beschriebenen Verfahren und Vorrichtungen zumindest weitgehend vermeiden. Insbesondere sollen ein Verfahren und eine Sterilisationsvorrichtung bereitgestellt werden, welche auf einfache Weise gehandhabt werden können, welche dennoch eine vollumfängliche Sterilisation des gesamten Sensors ermöglichen und welche auch in großtechnischem Maßstab einer Serienfertigung von Sensoren einsetzbar sind.

### Offenbarung der Erfindung

Diese Aufgabe wird durch ein Verfahren und eine Sterilisationsvorrichtung zum Sterilisieren eines implantierbaren Sensors zur Erfassung mindestens eines Analyten in einem Körpergewebe gemäß den unabhängigen Patentansprüchen gelöst. Vorteilhafte Weiterbildungen der Erfindung, welche einzeln oder in beliebiger Kombination realisierbar sind, sind in den abhängigen Patentansprüchen dargestellt.

Das Verfahren kann dabei unter Verwendung der erfindungsgemäßen Sterilisationsvorrichtung durchgeführt werden. Andererseits kann die Sterilisationsvorrichtung eingerichtet sein, um ein erfindungsgemäßes Verfahren durchzuführen. Dementsprechend kann für mögliche Einzelheiten des Verfahrens auf die Beschreibung der Sterilisationsvorrichtung verwiesen werden. Andererseits kann bezüglich möglicher Einzelheiten der Sterilisationsvorrichtung auf die Merkmale des Verfahrens verwiesen werden, so dass die Sterilisationsvorrichtung durch entsprechende Vorrichtungen eingerichtet sein kann, um das Verfahren durchzuführen und/oder um in dem Verfahren eingesetzt zu werden. Auch andere Ausgestaltungen des Verfahrens und/oder andere Ausgestaltungen der Sterilisationsvorrichtung sind jedoch grundsätzlich möglich.

In einem ersten Aspekt der vorliegenden Erfindung wird ein Verfahren zum Sterilisieren eines implantierbaren Sensors zur Erfassung mindestens eines Analyten in einem Körpergewebe vorgeschlagen. Unter einem implantierbaren Sensor wird allgemein ein Sensor verstanden, welcher vollständig oder teilweise in ein Körpergewebe eines menschlichen oder tierischen Benutzers einbringbar ist. Unter einem Sensor zur Erfassung mindestens eines Analyten in einem Körpergewebe wird allgemein eine Vorrichtung verstanden, welche eingerichtet ist, um qualitativ oder quantitativ das Vorhandensein des mindestens einen Analyten in dem Körpergewebe nachzuweisen. Bei dem mindestens einen Analyten kann es sich beispielsweise um einen Metaboliten und/oder eine andere Substanz handeln, welche in einem menschlichen oder tierischen Körpergewebe in unterschiedlichen Konzentrationen vorhanden sein kann, beispielsweise in einer in dem Körpergewebe enthaltenen Körperflüssigkeit. Insbesondere kann es sich bei dem mindestens einen Analyten um einen Analyten handeln, ausgewählt aus der Gruppe bestehend aus Blutglucose, Laktat, Cholesterin. Auch andere Analyte sind jedoch grundsätzlich nachweisbar.

Bei einer Sterilisation handelt es sich grundsätzlich um ein Verfahren, bei welchem Keime abgetötet werden. Insbesondere können diese Keime vollständig abgetötet werden, so dass nach dem Sterilisationsverfahren eine Vermehrung von Keimen nicht mehr möglich ist. Wie unten aufgeführt, kann die Sterilisation insbesondere eine Strahlensterilisation mit ionisierender Strahlung umfassen, besonders bevorzugt mit Partikelstrahlung und insbesondere mittels β -Strahlung und/oder Elektronenstrahlung. Die Strahlung kann beispielsweise eine Elektronenstrahlung mit einer Energie von 1.0 MeV bis 10 MeV umfassen kann, insbesondere von 2 MeV bis 3 MeV und besonders bevorzugt von 2,5 MeV.

Der Sensor weist mindestens einen in das Körpergewebe einbringbaren Sensorteil mit mindestens einer Sensorelektrode zur Erfassung des Analyten und mindestens einen Elektronikteil auf. Der Elektronikteil weist mindestens ein elektronisches Bauelement auf und ist mit dem Sensorteil verbunden. Diese Verbindung kann insbesondere eine mechanische Verbindung sein oder eine mechanische Verbindung umfassen. Alternativ oder vorzugsweise zusätzlich kann diese Verbindung zwischen dem Elektronikteil und dem Sensorteil auch mindestens eine elektrische Verbindung umfassen, beispielsweise mindestens eine elektrische Verbindung zwischen mindestens einem elektronischen Bauelement des Elektronikteils und mindestens einer Sensorelektrode des Sensorteils, beispielsweise über mindestens eine elektrische Zuleitung. Allgemein kann der Elektronikteil also vorzugsweise mechanisch und elektrisch mit dem Sensorteil verbunden sein.

Der Sensorteil kann vollständig oder teilweise in das Körpergewebe des Benutzers implantiert werden. Beispielsweise kann der Sensorteil flexibel ausgestattet sein. Beispielsweise kann der Sensorteil vollständig oder teilweise als Sensorstreifen ausgestaltet sein, mit einem flexiblen Substrat, welches in dem Körpergewebe aufnehmbar ist. Auch andere Ausgestaltungen sind jedoch grundsätzlich möglich.

Die mindestens eine Sensorelektrode ist derart eingerichtet, dass der Sensor den mindestens einen Analyten auf elektrochemischem Wege erfassen kann. So kann die mindestens eine Sensorelektrode insbesondere mindestens eine Arbeitselektrode und mindestens eine weitere Elektrode umfassen. Die mindestens eine Arbeitselektrode kann beispielsweise mindestens eine leitfähige Elektrodenschicht umfassen, welche mit mindestens einem Enzym beschichtet ist, sowie ggf. einem oder mehreren Zusatzstoffen. Bei dem mindestens einen Enzym kann es sich beispielsweise um ein Enzym handeln, welches auf den nachzuweisenden Analyten angepasst ist. Beispielsweise kann das mindestens eine Enzym Glucoseoxidase und/oder Glucosedehydrogenase umfassen. Auch andere Arten von Enzymen sind jedoch grundsätzlich möglich. Weiterhin kann eine Beschichtung der Sensorelektrode einen oder mehrere Mediatoren und/oder einen oder mehrere weitere Hilfsstoffe zum elektrochemischen Nachweis des mindestens einen Analyten umfassen. Die mindestens eine weitere Elektrode kann vorzugsweise mindestens eine Referenzelektrode und/oder mindestens eine Gegenelektrode umfassen. Beispielsweise kann die mindestens eine Referenzelektrode eine Ag/AgCl-Elektrode umfassen. Die mindestens eine Gegenelektrode kann beispielsweise eine Metallelektrode, bevorzugt aus einem Edelmetall, eine Carbonelektrode oder eine Elektrode aus einem elektrisch leitendem Polymer umfassen. Auch andere Ausgestaltungen der Elektroden sind jedoch grundsätzlich möglich. Insbesondere kann die mindestens eine Elektrode somit zwei, drei oder mehr Elektroden umfassen.

Das Sensorteil ist mit dem Elektronikteil verbunden. Insbesondere können der Sensorteil und der Elektronikteil gemeinsam eine Einheit bilden, welche vorzugsweise ohne Zerstörung des Sensors nicht trennbar ist. Beispielsweise können der Sensorteil und der Elektronikteil mindestens ein Substrat teilen. Die Verbindung kann auch eine lösbare oder unlösbare Klebeverbindung oder Klemmverbindung umfassen, welche die mechanische und elektrische Verbindung zwischen dem Sensorteil und dem Elektronikteil sicherstellt.

Der Sensorteil kann beispielsweise einen Schichtaufbau aufweisen. Insbesondere kann der Sensorteil mindestens ein Kunststoffsubstrat umfassen, beispielsweise ein Polyester- oder ein Polyimid-Substrat. Auch andere Ausgestaltungen sind möglich.

Der Elektronikteil ist vorzugsweise während der Erfassung des mindestens einen Analyten in dem Körpergewebe außerhalb des Körpergewebes angeordnet. Der Elektronikteil weist das mindestens eine elektronische Bauelement auf. Dieses mindestens eine elektronische Bauelement kann über die Verbindung zwischen dem Sensorteil und dem Elektronikteil insbesondere direkt oder indirekt mit der mindestens einen Elektrode verbunden sein. Insbesondere kann das mindestens eine elektronische Bauelement mindestens ein aktives Halbleiterbauelement umfassen. Besonders bevorzugt kann das elektronische Bauelement mindestens ein Verstärkerbauelement umfassen, vorzugsweise ein hochohmiges Verstärkerbauelement mit einem Eingangswiderstand von mindestens 1 GΩ, vorzugsweise von mindestens 100 GΩ. Insbesondere kann das mindestens eine elektronische Bauelement einen Potentiostaten umfassen.

Der Elektronikteil kann beispielsweise seinerseits mindestens ein Substrat aufweisen, vorzugsweise ein flexibles Substrat. Dieses Substrat kann beispielsweise einen Schaltungsträger umfassen, auf welchen das mindestens eine Bauelement aufgebracht ist. Das Substrat des Elektronikteils kann beispielsweise mit dem Substrat des Sensors verbunden sein. Weiterhin kann der Elektronikteil durch eine oder mehrere Zuleitungen mit der mindestens einen Elektrode des Sensorteils verbunden sein.

Das Verfahren weist folgende Schritte auf, welche vorzugsweise in der genannten Reihenfolge durchgeführt werden. Auch eine andere Reihenfolge ist grundsätzlich möglich. Weiterhin können auch einzelne oder mehrere Verfahrensschritte zeitlich parallel oder zeitlich überlappend durchgeführt werden. Weiterhin können einzelne oder mehrere der im Folgenden beschriebenen Verfahrensschritte wiederholt durchgeführt werden. Weiterhin kann das Verfahren einen oder mehrere zusätzliche, nicht genannte Schritte aufweisen.

In einem Verfahrensschritt (Verfahrensschritt a)) wird der Sensor in mindestens eine Verpackung eingebracht. Unter einem Einbringen wird dabei allgemein eine Bereitstellung des mindestens einen Sensors für das Verfahren in der Verpackung verstanden. Der Sensor kann sich dabei in einem fertigen Zustand befinden oder auch, wie nachfolgend noch näher beschrieben wird, in einem Halbfertigzustand, wobei einer oder mehrere nachfolgende Verfahrensschritte noch erforderlich sein können, um den halbfertigen Sensor in einen fertigen, am Benutzer einsetzbaren Zustand zu bringen. Beide Optionen sind im Rahmen des Begriffs der Einbringung des Sensors in die Verpackung umfasst.

Unter einer Verpackung wird allgemein im Rahmen der vorliegenden Erfindung eine Vorrichtung verstanden, welche den Sensor umschließt, vorzugsweise vollständig umschließt, und den Sensor gegenüber Umwelteinflüssen abschließt. So schließt die Verpackung den Sensor keimdicht gegen eine Umgebung ab, so dass durch die Verpackung keine Keime zu dem Sensor gelangen können. Die Verpackung ist dabei allgemein nicht als Bestandteil des Sensors anzusehen und ist allgemein derart ausgestaltet, dass diese für eine Verwendung des Sensors von dem Sensor getrennt wird. So kann der Sensor insbesondere beweglich in der Verpackung aufgenommen sein. Insbesondere kann die Verpackung vorzugsweise nicht mit dem Sensor verbunden sein. Die Verpackung kann beispielsweise ganz oder teilweise aus einem Kunststoffmaterial und/oder ganz oder teilweise aus einem metallischen Material hergestellt sein. Besonders bevorzugt umfasst die Verpackung mindestens eine Folie, vorzugsweise mindestens eine Kunststofffolie. Beispielsweise kann die Kunststofffolie mindestens einen der folgenden Kunststoffe umfassen: PE, PP,PC, PET, PETG. Auch andere Kunststoffe sind jedoch grundsätzlich alternativ oder zusätzlich einsetzbar. Weiterhin sind, alternativ oder zusätzlich, auch metallische Folien und/oder Verbundwerkstoff-Folien einsetzbar. Die Verpackung kann insbesondere verformbar, insbesondere flexibel, ausgestaltet sein. Alternativ oder zusätzlich kann die Verpackung jedoch auch ganz oder teilweise starr ausgestaltet sein. Die Verpackung kann, alternativ oder zusätzlich zu einer Folie, weiterhin auch mindestens ein Formteil umfassen, beispielsweise mindestens ein Kunststoffformteil. So können beispielsweise Formteile eingesetzt werden, welche durch einen Spritzgießprozess, Spritzblasprozess oder ähnliche formgebende Prozesse hergestellt werden können.

Die Verpackung nimmt weiterhin einen den Elektronikteil abschirmenden Strahlenschirm auf. Unter einem Strahlenschirm ist dabei eine Vorrichtung zu verstehen, welche die Strahlung, welche für die Sterilisation verwendet wird, um mindestens einen Faktor 10 abschwächt, vorzugsweise um mindestens einen Faktor 100. Details zur möglichen Auslegung eines Strahlenschirms finden sich beispielsweise in: W. Demtröder: Experimentalphysik, Band 4: Kern-, Teilchen- und Astrophysik, Springer Verlag, Heidelberg 1998, S. 91 - 92. Beispielsweise kann der Strahlenschirm ein plattenförmiges Element und/oder ein Formteil aufweisen, welches bei einer gerichteten Bestrahlung mit sterilisierenden Strahlung von der sterilisierenden Strahlung zunächst passiert werden muss, bevor diese den Elektronikteil erreicht. Der Elektronikteil kann dabei vollständig oder teilweise durch den Strahlenschirm abgeschirmt werden, wobei zumindest das elektronische Bauelement und/oder zumindest eines der elektronischen Bauelemente, vorzugsweise das Halbleiterbauelement und besonders bevorzugt der mindestens eine hochohmige Verstärker und/oder der mindestens eine Potentiostat, durch den Strahlenschirm abgeschirmt wird.

Unter dem Merkmal, dass die Verpackung den Strahlenschirm aufnimmt, kann dabei einerseits verstanden werden, dass der Strahlenschirm Bestandteil der Verpackung ist. Andererseits kann der Strahlenschirm jedoch auch, wie unten noch näher ausgeführt wird, lose in und/oder an der Verpackung aufgenommen sein und beispielsweise von der Verpackung trennbar ausgestaltet werden. Insbesondere kann die Verpackung den Strahlenschirm ganz oder teilweise umhüllen. Beispielsweise kann der Strahlenschirm in einem Innenraum der Verpackung aufgenommen sein. Alternativ oder zusätzlich kann der Strahlenschirm jedoch auch ganz oder teilweise außerhalb eines Innenraums der Verpackung angeordnet sein, so dass zwischen dem Innenraum und dem Strahlenschirm mindestens eine Wand der Verpackung angeordnet ist. Beispielsweise kann der Strahlenschirm ganz oder teilweise in einer oder mehreren in den Innenraum hineinragenden Taschen der Verpackung angeordnet sein, so dass der Strahlenschirm in den Innenraum der Verpackung hineinragt, wobei jedoch vorzugsweise zwischen dem Innenraum und dem Strahlenschirm mindestens eine Wand der Verpackung angeordnet ist. Verschiedene Ausgestaltungen sind möglich und werden exemplarisch unten noch näher beschrieben. Sämtliche Optionen, also Optionen, bei welchen der Strahlenschirm innerhalb der Verpackung oder lediglich an der Verpackung angeordnet ist, sollen von dem Merkmal, dass die Verpackung den Strahlenschirm aufnimmt, umfasst sein. Vorzugsweise bilden die Verpackung und der Strahlenschirm eine Einheit, unabhängig davon, ob diese Einheit trennbar ist oder nicht.
In einem weiteren Verfahrensschritt (Verfahrensschritt b)), welcher vorzugsweise vollständig nach dem Verfahrensschritt a) durchgeführt wird, wird der Sensor in der Verpackung aus mindestens einer Bestrahlungsrichtung mit sterilisierender Strahlung bestrahlt. Beispielsweise kann der Sensor aus einer Bestrahlungsrichtung und/oder aus zwei Bestrahlungsrichtungen mit der sterilisierenden Strahlung bestrahlt werden. Diese Bestrahlung kann vorzugsweise vollständig erfolgen, so dass der gesamte Sensor durch die sterilisierende Strahlung bestrahlt wird, mit Ausnahme derjenigen Abschnitte, welche durch den Strahlenschirm abgeschirmt werden. Auch eine punktuelle Bestrahlung ist jedoch grundsätzlich möglich. Vorzugsweise erfolgt die Bestrahlung jedoch derart großflächig, dass zunächst sämtlich Bestandteile des Sensors bestrahlt werden, wobei der Strahlenschirm, wie oben beschrieben, einen oder mehrere Bereiche des Sensors vor der sterilisierenden Strahlung abschirmt.

Unter einer sterilisierende Strahlung ist allgemein eine ionisierende Strahlung zu verstehen, welche keimabtötende Wirkungen aufweist. Hierbei kann es sich grundsätzlich um elektromagnetische Strahlung und/oder um Partikelstrahlung handeln. Besonders bevorzugt ist jedoch, wie oben ausgeführt, die Verwendung von Partikelstrahlung, insbesondere von β -Strahlung und/oder Elektronenstrahlung. Beispielsweise kann der Sensor während der Strahlensterilisation mit der sterilisierenden Strahlung mit einer Dosis von mindestens 5 kGy, besonders bevorzugt von mindestens 10 kGy und insbesondere von mindestens 20 kGy oder sogar mindestens 25 kGy beaufschlagt werden. Für die Strahlensterilisation können beispielsweise eine oder mehrere Strahlenquellen verwendet werden, beispielsweise radioaktive β -Emitter und/oder Elektronenstrahlenquellen. Bei der Strahlensterilisation, also der Bestrahlung des Sensors mit der sterilisierenden Strahlung, welche vorzugsweise gerichtet aus mindestens einer, vorzugsweise mindestens zwei Raumrichtungen erfolgt, schirmt der Strahlenschirm das elektronische Bauelement des Elektronikteils gegenüber der sterilisierenden Strahlung ab. Sind mehrere elektronische Bauelemente enthalten, so kann der Strahlenschirm insbesondere mindestens eines dieser elektronischen Bauelemente abschirmen, beispielsweise mindestens ein Halbleiterbauelement, vorzugsweise ein aktives Halbleiterbauelement, insbesondere mindestens einen Verstärker, vorzugsweise mindestens einen hochohmigen Verstärker und besonders bevorzugt einen Potentiostaten. Weiterhin ist der Strahlenschirm derart angeordnet, dass der Sensorteil durch die sterilisierende Strahlung sterilisiert wird. Im Gegensatz zum oben beschriebenen Stand der Technik, in welchem in der Regel der Sensorteil abgeschirmt wird, wird vorliegend also vorgeschlagen, den Elektronikteil vollständig oder teilweise abzuschirmen, den zu implantierenden Sensorteil, welcher unmittelbar in das Körpergewebe eingeführt wird, jedoch zu sterilisieren. Die Strahlenschäden, welche möglicherweise dabei an der mindestens einen Sensorelektrode auftreten, beispielsweise an einer Sensorchemie der Sensorelektroden, beispielsweise dem mindestens einem Enzym, sind in der Regel vernachlässigbar oder lassen sich durch eine entsprechende Kalibration nach der Strahlensterilisation ausgleichen. Gleichzeitig werden jedoch empfindliche Halbleiterbauelemente, wie beispielsweise Potentiostaten, gegenüber der sterilisierenden Strahlung abgeschirmt, so dass die Strahlensterilisation für diese Bauelemente in der Regel mit keiner Funktionseinbuße verbunden ist. Auf diese Weise lassen sich insbesondere einstückig oder als eine Einheit ausgebildete Sensoren herstellen, bei welchen gezielt ein Elektronikteil vollständig oder teilweise vor Strahlenschäden geschützt wird. Der Sensor kann während und nach der Strahlensterilisation in der Verpackung verbleiben, welche vorzugsweise während der Strahlensterilisation vollständig geschlossen ist und welche beispielsweise nach der Strahlensterilisation eine Wiederverkeimung des Sensors verhindern kann. Der Sensor kann in der Verpackung an einen Endkunden ausgeliefert werden, wobei beispielsweise der Endkunde die Verpackung öffnet, um den Sensor vollständig oder teilweise zu implantieren. Beispielsweise kann für dieses Öffnen die Verpackung mindestens eine Sollbruchstelle aufweisen, beispielsweise eine Schwächung einer Wanddicke, entlang derer ein Aufreißen der Verpackung möglich ist. Es kann auch eine Öffnung mit einem Stopfen oder Schraubverschluss vorgesehen sein.

Wie oben ausgeführt, kann der Sensor in Verfahrensschritt a) innerhalb der Verpackung bereitgestellt werden. Der Sensor kann dabei, wie ebenfalls ausgeführt, als fertiger Sensor oder auch als Halbfertigprodukt, also als Zwischenprodukt, bereitgestellt werden. In letzterem Fall können eine oder mehrere Verfahrensschritte erforderlich sein, um den Sensor fertig zu montieren. Besonders bevorzugt wird das Verfahren derart durchgeführt, dass diese Fertigmontage innerhalb der Verpackung erfolgt, ohne dass die Verpackung geöffnet wird. Diese Fertigmontage kann insbesondere nach der Strahlensterilisation des Verfahrensschritts b) erfolgen. Nach dem Einbringen des Sensors in die Verpackung kann die Verpackung vorzugsweise geschlossen werden, insbesondere keimdicht, beispielsweise durch mindestens ein Verschlusselement und/oder durch einen Formschluss, beispielsweise eine Verschweißung. So können beispielsweise eine Folie der Verpackung und/oder ein Folienteil der Verpackung verschweißt werden.

So kann der Sensor insbesondere nach Durchführung des Verfahrensschritts a) mindestens einen ersten Teil und mindestens einen zweiten Teil aufweisen. Der erste Teil kann in Verfahrensschritt b) sterilisiert werden. Der zweite Teil kann hingegen in Verfahrensschritt b) durch den Strahlenschirm gegenüber der sterilisierenden Strahlung vollständig oder teilweise, d.h. vollständig oder beispielsweise bereichsweise, abgeschirmt werden. Nach Durchführung des Verfahrensschritts b), also nach Durchführung der Strahlensterilisation, können der erste Teil und der zweite Teil innerhalb der Verpackung verbunden werden, insbesondere ohne ein Öffnen der Verpackung.

Dies bedeutet, dass die Verpackung vorzugsweise derart ausgestaltet ist, dass ein Eingriff eines Benutzers von außen derart möglich ist, dass nach der Strahlensterilisation der erste Teil und der zweite Teil innerhalb der Verpackung miteinander verbunden werden können. Zu diesem Zweck kann die Verpackung beispielsweise derart verformbar ausgestaltet sein, dass die beschriebene Fertigmontage, bei welcher der erste Teil und der zweite Teil innerhalb der Verpackung miteinander verbunden werden, möglich ist.

So kann die Verpackung allgemein derart verformbar ausgestaltet sein, dass bei einer Verformung der Verpackung nach Durchführung des Verfahrensschritts b) der erste Teil und der zweite Teil derart zueinander bewegt werden, dass diese miteinander verbunden werden. Insbesondere können der erste Teil und der zweite Teil gegeneinander gepresst werden. Wie unten noch näher ausgeführt wird, kann dies dadurch bewerkstelligt werden, dass der erste Teil und der zweite Teil bei der Bewegung, die durch die Verformung durch die Verpackung ausgelöst wird, durch mindestens eine Engstelle geführt werden, wobei durch die Engstelle der erste Teil und der zweite Teil gegeneinander gepresst werden. Im Folgenden wird diese Ausgestaltung insbesondere unter Bezugnahme auf einen Schichtaufbau des Sensors beschrieben, wobei eine oder mehrere Schichten des Sensors als erster Teil sterilisiert werden, wobei eine oder mehrere weitere Schichten des Sensors als zweiter Teil während der Strahlensterilisation vollständig oder teilweise, d.h. insgesamt oder in mindestens einem Bereich, gegenüber der sterilisierenden Strahlung abgeschirmt werden. Auch andere Ausgestaltungen der Fertigmontage innerhalb der Verpackung sind jedoch grundsätzlich möglich, abgesehen von einem Schichtaufbau.

In einer bevorzugten Ausgestaltung des Verfahrens ist der Elektronikteil derart ausgestaltet, dass dieser als Ganzes oder in mindestens einem Bereich einen Schichtaufbau aufweist. Unter einem Schichtaufbau ist allgemein ein Aufbau zu verstehen, bei welchem eine oder mehrere Schichten aufeinander aufgebracht werden. Mindestens eine dieser Schichten kann beispielsweise mindestens einen Schaltungsträger aufweisen, auf welchen das mindestens eine elektronische Bauelement des Elektronikteils aufgebracht ist und/oder in welchen das mindestens eine elektronische Bauelement des Elektronikteils eingebracht ist. Der Schichtaufbau weist mindestens eine Deckschicht auf. In dem Verfahrensschritt b) wird die Deckschicht dabei derart zu dem Strahlenschirm angeordnet, dass die Deckschicht zumindest teilweise durch die sterilisierende Strahlung sterilisiert wird. Unter einer Deckschicht ist dabei eine Schicht zu verstehen, welche in dem genannten Schichtaufbau mindestens eine weitere Schicht und/oder mindestens ein weiteres Bauelement, beispielsweise das elektronische Bauelement, überdeckt. Beispielsweise kann die Deckschicht bei einem fertigmontierten Sensor eine Schicht darstellen, welche eine Oberfläche des Sensors bildet und welche beispielsweise in Berührung mit einer Haut und/oder einer Körperflüssigkeit und/oder dem Körpergewebe eines Benutzers gelangen kann.

Die Deckschicht kann insbesondere eine Klebeschicht umfassen, insbesondere mindestens eine Klebeschicht zum Fixieren des implantierbaren Sensors auf einer Hautoberfläche. So kann der implantierbare Sensor insbesondere derart ausgestaltet sein, dass das Sensorteil in das Körpergewebe durch mindestens eine Insertionsöffnung hineinragt, wohingegen der Elektronikteil vollständig oder teilweise außerhalb des Körpergewebes angeordnet ist, beispielsweise auf einer Hautoberfläche eines Benutzers. Der Elektronikteil kann durch die genannte mindestens eine Klebeschicht, beispielsweise ein Pflaster, auf der Hautoberfläche des Benutzers fixierbar sein.

Das Verfahren kann weiterhin derart ausgestaltet sein, dass, wie oben beschrieben, nach der Strahlensterilisation die Fertigmontage erfolgt, bei welcher die Deckschicht auf eine oder mehrere weitere Schichten aufgebracht wird. Insbesondere kann nach Durchführung des Verfahrensschritts b) die Deckschicht, welche nunmehr sterilisiert ist, innerhalb der Verpackung auf das elektronische Bauelement aufgebracht werden. Dieses Aufbringen kann direkt oder indirekt erfolgen, also derart, dass die Deckschicht das mindestens eine elektronische Bauelement unmittelbar berührt, oder indirekt, indem eine oder mehrere Zwischenlagen zwischen der Deckschicht und dem elektronischen Bauelement aufgebracht werden, beispielsweise eine oder mehrere weitere Schichten und/oder eine oder mehrere Verkapselungen. Verschiedene Ausgestaltungen sind möglich. In jedem Fall kann das Aufbringen derart erfolgen, dass zwischen dem elektronischen Bauelement und einer äußeren Oberfläche des Sensors die mindestens eine Deckschicht angeordnet ist. Das Aufbringen der Deckschicht auf das elektronische Bauelement kann insbesondere derart erfolgen, dass während dieses Aufbringens die Verpackung nicht geöffnet wird. Während der Fertigmontage kann der Sensor also durch die Verpackung noch vollständig vor einer Wiederverkeimung geschützt werden.

Nach dem Aufbringen der Deckschicht kann die Deckschicht insbesondere, wie oben ausgeführt, eine äußere Oberfläche des Sensors, insbesondere des Elektronikteils des Sensors, bilden. Dies kann insbesondere derart ausgestaltet werden, dass die äußere Oberfläche des Elektronikteils, besonders bevorzugt die gesamte Oberfläche des implantierbaren Sensors, vollumfänglich sterilisiert ist. In anderen Worten kann das Verfahren derart durchgeführt werden, dass auch im Bereich des Elektronikteils die Oberfläche des Sensors vollständig strahlensterilisiert ist, wobei dennoch das mindestens eine elektronische Bauelement durch Verwendung des Strahlenschirms und anschließende Fertigmontage innerhalb der Verpackung vor Strahlenschäden geschützt sein kann.

Die Verpackung kann, wie oben ausgeführt, insbesondere vollständig oder teilweise verformbar ausgestaltet sein. Durch eine Verformung kann die genannte Fertigmontage, bei welcher der erste Teil und der zweite Teil miteinander verbunden werden, insbesondere indem die Deckschicht auf das elektronische Bauelement aufgebracht wird, bewerkstelligt werden. So kann die Verpackung insbesondere derart verformbar ausgestaltet sein, dass bei einer Verformung der Verpackung der Sensor durch mindestens eine Engstelle bewegt wird, insbesondere gezogen wird, wobei durch die Engstelle die Deckschicht auf das elektronische Bauelement aufgepresst wird. Diese Engstelle kann auf verschiedene Weisen ausgestaltet sein und kann beispielsweise mindestens einen Spalt umfassen. Vorzugsweise ist dieser Spalt nicht vollständig starr ausgebildet, um Beschädigungen des Sensors zu vermeiden. Auf diese Weise kann beispielsweise ein Laminierverfahren erfolgen, wobei die Deckschicht auf das mindestens eine elektronische Bauelement auf laminiert wird. Beispielsweise kann die Engstelle durch mindestens ein Druckkissen in der Verpackung gebildet werden, welches mit einem Gegenelement zusammenwirkt, beispielsweise einem starren Gegenelement und/oder einem weiteren Druckkissen, wobei sich zwischen dem Druckkissen und dem Gegenelement ein Spalt ausbilden kann.

Die Verformung der Verpackung kann insbesondere durch Verwendung eines flexiblen Verpackungsmaterials erfolgen, welches dehnbar und/oder verformbar ist. Alternativ oder zusätzlich kann die Verpackung auch beispielsweise gezielt verformbare Elemente aufweisen, wie beispielsweise mindestens einen Faltenbalg.

Wie oben ausgeführt, kann der Strahlenschirm Bestandteil der Verpackung sein oder kann jedoch lediglich mit der Verpackung verbunden sein und/oder in der Verpackung und/oder an der Verpackung aufgenommen sein, so dass der Strahlenschirm selbst keinen Bestandteil der eigentlichen Verpackung bildet. In letzterem Fall kann der Strahlenschirm insbesondere nach Durchführung des Verfahrensschritts b) von der Verpackung getrennt werden, um beispielsweise für die Sterilisation weiterer Sensoren eingesetzt zu werden. So kann der Strahlenschirm insbesondere als wiederverwendbarer Strahlenschirm ausgestaltet sein.

Diese Trennung des Strahlenschirms von der Verpackung kann insbesondere eine Entfernung des Strahlenschirms aus einer vollständigen oder teilweisen Umhüllung des Strahlenschirms durch die Verpackung beinhalten. Die Trennung kann insbesondere gleichzeitig oder zumindest zeitlich überlappend oder in einem Vorgang mit der oben beschriebenen Verformung der Verpackung erfolgen. So kann bei der Verformung der Verpackung gleichzeitig der Strahlenschirm von der Verpackung getrennt werden, insbesondere ganz oder teilweise aus einer vollständigen oder teilweisen Umhüllung durch die Verpackung entfernt werden. Beispielsweise kann der Strahlenschirm vor dem Verformen der Verpackung von außen in mindestens einen in einen Innenraum der Verpackung hineinragenden Finger der Verpackung eingesteckt sein. Beispielsweise kann die Verpackung allgemein einen Innenraum bilden, in welchem der Sensor während der Strahlensterilisation und vorzugsweise während der Fertigmontage aufgenommen ist und keimdicht gelagert ist. Unter einem Finger ist allgemein ein Vorsprung einer Innenwand der Verpackung zu verstehen, welcher in den Innenraum hineinragt. Beispielsweise kann der Finger mindestens eine in den Innenraum ragende Tasche bilden oder umfassen. Dieser Finger kann röhrenförmig ausgestaltet sein und/oder auf andere Weise ausgestaltet sein, derart, dass der Strahlenschirm ebenfalls in den Innenraum hineinragt und während der Strahlensterilisation das mindestens eine elektronische Bauelement des Elektronikteils abschirmen kann. Allgemein kann also der Strahlenschirm derart angeordnet werden, dass dieser nicht in dem Innenraum aufgenommen ist, so dass der Strahlenschirm beispielsweise selbst nicht steril sein muss und beispielsweise wiederverwendet werden kann, ohne dass eine Sterilisation des Strahlenschirms erforderlich wäre.

Das Verfahren kann insbesondere derart durchgeführt werden, dass der Finger der Verpackung, welcher während der Strahlensterilisation in die Verpackung hineinragt, insbesondere in den Innenraum der Verpackung, beim Entfernen des Strahlenschirms aus einem Innenraum der Verpackung herausgezogen wird. Dies kann beispielsweise in gleicher Weise erfolgen, wie die Finger eines Handschuhs beim Herausziehen der Hand umgestülpt werden können. Beispiele werden unten noch näher beschrieben.

Die Verpackung kann insbesondere derart verformbar ausgestaltet sein, dass der Sensor mit einem ersten Verpackungsteil der Verpackung verbunden ist, beispielsweise durch eine lösbare Verbindung, wobei der Strahlenschirm mit einem zweiten Verpackungsteil der Verpackung bewegbar ist, wobei während der Verformung der Verpackung der erste Verpackungsteil und der zweite Verpackungsteil derart zueinander verbunden bleiben, dass eine keimdichte Abschirmung des Sensors weiterhin gewährt ist, beispielsweise bis zu einem Öffnen der Verpackung durch einen Benutzer zum Zweck einer Entnahme des Sensors aus der Verpackung.

Bei der Verformung der Verpackung kann die Deckschicht großflächig von mindestens einer Seite auf ein das elektronische Bauelement tragende Substrat des Elektronikteils aufgebracht werden, insbesondere aufgeklebt und/oder auf laminiert werden. Grundsätzlich sind, wie oben beschrieben, jedoch auch andere Arten der Fertigmontage möglich.

Die Verpackung kann weiterhin mindestens einen Griff, vorzugsweise mindestens zwei Griffe zum Halten der Verpackung durch einen die Verformung durchführenden Nutzer aufweisen. Beispielsweise kann mindestens ein erster Griff mit einem oben beschriebenen ersten Verpackungsteil verbunden sein, und mindestens ein zweiter Griff mit mindestens einem zweiten Verpackungsteil.

Der Strahlenschirm kann insbesondere mindestens einen metallischen Strahlenschirm umfassen. Der metallische Strahlenschirm kann insbesondere in eine Dicke von 1 mm bis 10 mm aufweisen, vorzugsweise eine Dicke von 3 mm bis 7 mm und besonders bevorzugt eine Dicke von 5 mm. Auch andere Ausgestaltungen sind jedoch grundsätzlich möglich. Der metallische Strahlenschirm kann insbesondere vollständig oder teilweise hergestellt sein aus mindestens einem metallischen Material, ausgewählt aus der Gruppe bestehend aus: Aluminium, Eisen, Stahl, Blei, Kupfer. Alternativ oder zusätzlich ist jedoch auch die Verwendung anderer Metalle möglich. Die genannten Metalle und/oder andere Metalle können in Reinform vorliegen und/oder in Form von Legierungen.

Zusätzlich zu dem Sensor kann weiterhin mindestens ein weiteres Element in der Verpackung aufgenommen sein und bei der Strahlensterilisation vorzugsweise mit sterilisiert werden. So kann beispielsweise in dem Verfahrensschritt a) zusätzlich mindestens ein medizinisches Hilfsmittel in die Verpackung eingebracht werden, d.h. in der Verpackung bereitgestellt werden. Unter einem medizinischen Hilfsmittel ist allgemein eine Vorrichtung zu verstehen, welche im Rahmen eines medizinischen Prozesses eingesetzt werden kann, also im Rahmen eines diagnostischen und/oder chirurgischen und/oder therapeutischen Prozesses. Insbesondere kann es sich bei dem medizinischen Hilfsmittel um ein Einweg-Hilfsmittel handeln. Beispielsweise kann es sich bei dem medizinischen Hilfsmittel um eine Vorrichtung handeln, welche mit einer Körperflüssigkeit und/oder einem offenen Körpergewebe eines Benutzers in Kontakt kommen kann. Insbesondere kann das medizinische Hilfsmittel mindestens eine Insertionshilfe umfassen. Unter einer Insertionshilfe ist beispielsweise ein Element zu verstehen, welches eingerichtet ist, um zumindest den Sensorteil des Sensors in das Körpergewebe einzuführen. Beispielsweise kann die Insertionshilfe mindestens eine Kanüle umfassen. Bezüglich möglicher Ausgestaltungen des implantierbaren Sensors sowie bezüglich möglicher Ausgestaltungen der Insertionshilfe kann grundsätzlich auf die obige Beschreibung des Standes der Technik verwiesen werden. Auch andere Ausgestaltungen sind jedoch grundsätzlich möglich.

In einem weiteren Aspekt der vorliegenden Erfindung wird, wie oben ausgeführt, eine Sterilisationsvorrichtung zum Sterilisieren eines implantierbaren Sensors zur Erfassung mindestens eines Analyten in einem Körpergewebe vorgeschlagen. Unter einer Sterilisationsvorrichtung ist dabei allgemein eine Vorrichtung zu verstehen, welche den Sensor während und vorzugsweise auch nach der Strahlensterilisation aufnehmen kann. Die Sterilisationsvorrichtung kann beispielsweise als Ganze reversibel in eine Sterilisationsapparatur eingefügt werden, welche beispielsweise eine Strahlenquelle zum Erzeugen der sterilisierenden Strahlung aufweist, beispielsweise eine β - Strahlenquelle und/oder eine Elektronenstrahlenquelle. Die Sterilisationsvorrichtung kann beispielsweise eine Einheit bilden, welche als Ganze gehandhabt werden kann und welche beispielsweise vollständig oder teilweise später an einen Endkunden oder Benutzer ausgeliefert werden kann.

Die Sterilisationsvorrichtung dient insbesondere zum Einsatz in einem Verfahren gemäß einem oder mehreren der oben beschriebenen Ausgestaltungen und/oder gemäß einer oder mehreren der nachfolgend noch näher beschriebenen Ausführungsbeispiele.

Die Sterilisationsvorrichtung umfasst mindestens einen implantierbaren Sensor zur Erfassung mindestens eines Analyten in einem Körpergewebe. Der Sensor weist mindestens einen in das Körpergewebe einbringbaren Sensorteil mit mindestens einer Sensorelektrode zur Erfassung des Analyten sowie mindestens einen Elektronikteil auf. Der Elektronikteil weist mindestens ein elektronisches Bauelement auf und ist mit dem Sensorteil verbunden. Bezüglich weiterer möglicher Ausgestaltungen des Sensors kann auf die obige Beschreibung verwiesen werden.

Weiterhin umfasst die Sterilisationsvorrichtung mindestens eine Verpackung. Die Verpackung schließt den Sensor keimdicht ab. Die Verpackung nimmt mindestens einen das elektronische Bauelement des Elektronikteils während einer Strahlensterilisation abschirmenden Strahlenschirm auf. Wie oben beschrieben, kann der Strahlenschirm Bestandteil der Verpackung sein oder kann auch lösbar in der Verpackung aufgenommen sein, d.h. mit der Verpackung derart verbunden sein, dass der Strahlenschirm nach der Strahlensterilisation von der Verpackung getrennt werden kann. Bezüglich weiterer möglicher Ausgestaltungen kann auf die obige Beschreibung verwiesen werden.

Die Strahlensterilisation kann insbesondere derart eingerichtet sein, dass diese während eines erfindungsgemäßen Verfahrens einsetzbar ist.

So kann der Sensor insbesondere mindestens einen ersten Teil und mindestens einen zweiten Teil aufweisen, wobei der erste Teil bei der Strahlensterilisation in der Verpackung sterilisierbar ist, wobei der zweite Teil bei der Strahlensterilisation durch den Strahlenschirm abgeschirmt wird, wobei nach Durchführung der Strahlensterilisation der erste Teil und der zweite Teil innerhalb der Verpackung verbindbar sind, insbesondere ohne Öffnen der Verpackung. Die Sterilisationsvorrichtung kann also derart eingerichtet sein, dass innerhalb der Verpackung nach der Strahlensterilisation eine Fertigmontage des Sensors durchführbar ist.

Insbesondere kann, wie oben ausgeführt, die Verpackung verformbar ausgestaltet sein. Bei einer Verformung der Verpackung nach Durchführung der Strahlensterilisation können der erste Teil und der zweite Teil derart zueinander bewegbar sein, dass diese miteinander verbunden werden. Insbesondere können der erste Teil und der zweite Teil gegeneinander gepresst werden.

Der Elektronikteil kann, wie oben ausgeführt, insbesondere mindestens einen Schichtaufbau aufweisen. Der Schichtaufbau kann insbesondere mindestens eine Deckschicht aufweisen, wobei die Verpackung derart ausgestaltet sein kann, dass während der Strahlensterilisation die Deckschicht derart zu dem Strahlenschirm angeordnet ist, dass die Deckschicht zumindest teilweise durch die sterilisierende Strahlung sterilisierbar ist, während das elektronische Bauelement abgeschirmt ist. Die Verpackung kann derart ausgestaltet sein, dass nach Durchführung der Strahlensterilisation die Deckschicht innerhalb der Verpackung auf das elektronische Bauelement aufgebracht werden kann, insbesondere aufgepresst und/oder auf laminiert werden kann, insbesondere ohne Öffnen der Verpackung. Wie oben ausgeführt, kann die Verpackung insbesondere derart verformbar ausgestaltet sein, dass bei einer Verformung der Verpackung der Sensor durch mindestens eine Engstelle bewegt wird, insbesondere gezogen wird, wobei durch die Engstelle die Deckschicht auf das elektronische Bauelement aufpressbar ist. Die Engstelle kann insbesondere durch mindestens ein Druckkissen in der Verpackung gebildet werden.

Die Verpackung kann insbesondere derart ausgestaltet sein, dass bei der Verformung gleichzeitig der Strahlenschirm von der Verpackung trennbar ist, insbesondere ganz oder teilweise aus einer vollständigen oder teilweisen Umhüllung durch die Verpackung entfernbar ist. So kann der Strahlenschirm beispielsweise vor dem Verformen der Verpackung von außen in mindestens einen in einen Innenraum der Verpackung hineinragenden Finger der Verpackung eingesteckt sein. Der Finger der Verpackung kann beim Entfernen des Strahlenschirms vorzugsweise aus dem Innenraum der Verpackung herausgezogen werden. Für weitere mögliche Ausgestaltungen kann auf die obige Beschreibung verwiesen werden.

Die Verpackung kann insbesondere derart verformbar ausgestaltet sein, dass der Sensor mit einem ersten Verpackungsteil der Verpackung verbunden ist, wobei der Strahlenschirm mit einem zweiten Verpackungsteil der Verpackung bewegbar ist, wobei während der Verformung der Verpackung der erste Verpackungsteil und der zweite Verpackungsteil derart miteinander verbunden bleiben, dass eine keimdichte Abschirmung des Sensors während der Verformung gewährleistet ist.

Die Verpackung kann insbesondere, wie oben beschrieben, mindestens einen Faltenbalg aufweisen. Beispielsweise können der erste Verpackungsteil und der zweite Verpackungsteil durch den Faltenbalg miteinander verbunden sein. Die Verpackung kann weiterhin mindestens zwei Griffe aufweisen, beispielsweise einen ersten, mit dem ersten Verpackungsteil verbundenen Griff und einen zweiten, mit dem zweiten Verpackungsteil verbundenen Griff. Das Vorsehen dieser Griffe kann eine Verformung der Verpackung erleichtern.

Die Verpackung kann weiterhin, wie oben beschrieben, insbesondere mindestens eine Folie, vorzugsweise mindestens eine Kunststofffolie und/oder mindestens einem Metallfolie und/oder mindestens eine Laminatfolie, aufweisen. Die Folie kann besonders in einem Bereich der Verpackung, der bei der Sterilisation von der sterilisierenden Strahlung durchstrahlt wird, angebracht sein. Beispielsweise kann diese Folie eine Dicke von maximal 1 mm aufweisen, vorzugsweise von maximal 500 µm und besonders bevorzugt eine Dicke von 10 µm bis 100 µm. Insbesondere kann die Verpackung einen oder mehrere der oben beschriebenen Kunststoffe aufweisen.

Die Verpackung kann vor oder nach einer Sterilisation durch an sich bekannte Druck- oder Vakuumverfahren auf Gas- und/oder Sterildichtigkeit überprüft werden.

Wie weiterhin oben ausgeführt, kann die Verpackung insbesondere mindestens eine Sollbruchstelle zum irreversiblen Öffnen und Entnehmen des Sensors aufweisen, insbesondere durch einen Endkunden und/oder Benutzer. So können beispielsweise die Verfahrensschritte a) und b) sowie ggf. eine Fertigmontage des Sensors innerhalb der Verpackung durch einen Hersteller erfolgen. Anschließend kann der Sensor gelagert und ausgeliefert werden, beispielsweise an einen Zwischenhändler und/oder einen Endkunden, wobei der Endkunde beispielsweise den Sensor nach einem Öffnen der Verpackung entnehmen kann, zum Insertieren des Sensors in das Körpergewebe. Die Öffnung erfolgt beispielsweise durch Aufreißen einer Sollbruchstelle, durch Entfernung eines Stopfens oder Schraubverschlusses. Die Sollbruchstelle kann beispielsweise mindestens eine Schwächung in der Verpackung umfassen, beispielsweise eine Materialschwächung in Form einer linienförmigen Schwächung, entlang derer die Verpackung aufgerissen und geöffnet werden kann. Auch andere Ausgestaltungen sind jedoch grundsätzlich möglich. Das Verfahren und die Sterilisationsvorrichtung gemäß einer oder mehreren der oben beschriebenen Ausgestaltungen weisen gegenüber bekannten Verfahren und Vorrichtungen zahlreiche Vorteile auf. Insbesondere kann nunmehr ein Sensor mit einem Elektronikteil und einem Sensorteil, der auch einstückig ausgebildet sein kann, problemlos sterilisiert werden. Insbesondere können beispielsweise marktübliche Potentiostate verwendet werden. Beispielsweise kann eine Strahlensterilisation mit 25 kGy ohne Funktionseinbuße überstanden werden.

Während die aus dem Stand der Technik bekannten Vorrichtungen und Verfahren nicht darauf eingerichtet sind, ein Sensorsystem zu sterilisieren, das ohne weitere komplizierte Schritte an einem menschlichen und/oder tierischen Körper angewendet werden kann, ist dies gemäß der vorliegenden Erfindung problemlos möglich. Insbesondere kann ein Sensor nunmehr die Haut und/oder das Körpergewebe und/oder eine Körperflüssigkeit berührende Teile aufweisen, die einerseits steril sein müssen, andererseits mit der empfindlichen Elektronik aber in direktem Kontakt stehen. Das Sensorsystem des Sensors kann nunmehr in komplett notwendigen Umfang sterilisiert werden, beispielsweise ohne dass mit verschiedenen Sterilisationsverfahren gearbeitet werden muss. So kann beispielsweise erfindungsgemäß ausschließlich eine Strahlensterilisation eingesetzt werden, ohne dass beispielsweise eine zusätzliche chemische Sterilisation erforderlich wäre.

Weiterhin können zusätzliche Arbeitsschritte bei der Fertigung und zusätzliche Handhabungsschritte bei der Anwendung eingespart werden. So kann eine Sensoreinheit hergestellt werden, welche in vollständig sterilem Zustand fertigmontiert wird, ohne dass anschließend bei einem Benutzer weitere Schritte erforderlich wären, mit Ausnahme der stets erforderlichen Insertion in das Körpergewebe.

Der implantierbare Sensorteil, welcher subkutan implantiert werden kann, ist mit dem Elektronikteil verbunden. Der Elektronikteil kann vollständig oder teilweise als Ansteuer- und Auswertungsteil des Sensors wirken. So kann der Elektronikteil beispielsweise eingerichtet sein, um eine Signalverarbeitung durchzuführen und/oder eine Vorverarbeitung von Signalen. Weiterhin kann der Elektronikteil auch als Kommunikationsteil eingerichtet sein und kann beispielsweise eine oder mehrere Schnittstellen des Sensors umfassen, mittels derer der Sensor beispielsweise Messdaten an einen Benutzer und/oder an eine oder mehrere weitere Vorrichtungen weitergeben kann. Weiterhin kann der Elektronikteil auch mindestens eine Energieversorgung umfassen, also beispielsweise eine eigenständige Energieversorgung und/oder einen Energiespeicher und/oder mindestens einen Anschluss, über welchen eine Energieversorgung erfolgen kann. Der implantierbare Sensorteil und alle anderen, die Haut berührenden Teile können durch β-Strahlung sterilisiert werden. Die Elektronik kann vollständig oder teilweise durch den Strahlenschirm, bevorzugt eine metallische Abschirmung, geschützt werden. Der Strahlenschirm kann optional aus der Verpackung entfernt werden, wobei bei diesem Schritt vorzugsweise eine Fertigmontage erfolgen kann, beispielsweise eine Verbindung zwischen dem vorher abgeschirmten Teil und einer Fixierungseinrichtung zur Fixierung des Teils auf der menschlichen Haut. So kann der oben genannte erste Teil, welcher im Verfahrensschritt b) sterilisiert wird, eine derartige Fixierungseinrichtung zur Fixierung des Sensors auf der menschlichen Haut, umfassen, beispielsweise mindestens eine Klebeschicht.

Allgemein kann trotz der notwendigen Sterilisation, beispielsweise mit energiereichen Strahlen, eine handelsübliche Elektronik in dem Elektronikteil des Sensors verwendet werden. So können beispielsweise handelsübliche Elektronikkomponenten, insbesondere in Form von Halbleiterbauelementen, eingesetzt werden, ohne dass Strahlenschäden zu befürchten sind.

Als weiterer Vorteil ist zu bemerken, dass durch die Option der Entnahme des Strahlenschirms das Sensorsystem bei der Anwendung am Patienten ein niedriges Profil aufweisen kann. So kann insbesondere durch Entnahme des Strahlenschirms die Verpackung mit dem darin aufgenommenen Sensor äußert kleinvolumig ausgestaltet werden, so dass die Einheit aus Verpackung und Sensor an einen Endkunden ausgeliefert werden kann. So können die Kosten und das Gewicht des Strahlenschirms eingespart werden. Vorteilhaft ist weiterhin, dass verschiedene Komponenten, wie beispielsweise eine Sensorspitze, eine Schlitzkanüle und eine die Haut berührender Klebefilm, gemeinsam in einer steril dichten Verpackung, welche als Umverpackung dienen kann, sterilisiert werden können. Nach der Sterilisation können diese Komponenten ohne Öffnung der als Sterilverpackung wirkenden Verpackung montiert werden und/oder in eine neue Relativposition zueinander gebracht werden.

Zusammenfassend sind im Rahmen der vorliegenden Erfindung folgende Ausführungsformen besonders bevorzugt:

Ausführungsform 1: Verfahren zum Sterilisieren eines implantierbaren Sensors zur Erfassung mindestens eines Analyten in einem Körpergewebe, wobei der implantierbare Sensor mindestens einen in das Körpergewebe einbringbaren Sensorteil mit mindestens einer Sensorelektrode zur Erfassung des Analyten und mindestens einen Elektronikteil aufweist, wobei der Elektronikteil mindestens ein elektronisches Bauelement aufweist und mit dem Sensorteil verbunden ist, wobei das Verfahren folgende Schritte aufweist:
a) der implantierbare Sensor wird in mindestens eine Verpackung eingebracht, wobei die Verpackung den implantierbaren Sensor keimdicht abschließt und wobei die Verpackung einen den Elektronikteil abschirmenden Strahlenschirm aufnimmt,
b) der implantierbare Sensor wird in der Verpackung aus mindestens einer Bestrahlungsrichtung mit sterilisierender Strahlung bestrahlt, insbesondere mit Elektronenstrahlung, wobei der Strahlenschirm das elektronische Bauelement des Elektronikteils gegenüber der sterilisierenden Strahlung abschirmt, wobei der Strahlenschirm derart angeordnet wird, dass der Sensorteil durch die sterilisierenden Strahlung sterilisiert wird.

Ausführungsform 2: Verfahren nach der vorhergehenden Ausführungsform, wobei der implantierbare Sensor nach Durchführung des Verfahrensschritts a) mindestens einen ersten Teil und mindestens einen zweiten Teil aufweist, wobei der erste Teil in Verfahrensschritt b) sterilisiert wird, wobei der zweite Teil in Verfahrensschritt b) durch den Strahlenschirm gegenüber der sterilisierenden Strahlung abgeschirmt wird, wobei nach Durchführung des Verfahrensschritts b) der erste Teil und der zweite Teil innerhalb der Verpackung verbunden werden, insbesondere ohne Öffnen der Verpackung.

Ausführungsform 3: Verfahren nach der vorhergehenden Ausführungsform, wobei die Verpackung verformbar ausgestaltet ist, wobei bei einer Verformung der Verpackung nach Durchführung des Verfahrensschritts b) der erste Teil und der zweite Teil derart zueinander bewegt werden, dass diese miteinander verbunden werden, insbesondere indem der erste Teil und der zweite Teil gegeneinander gepresst werden.

Ausführungsform 4: Verfahren nach einer der vorhergehenden Ausführungsformen, wobei der Elektronikteil einen Schichtaufbau aufweist, wobei der Schichtaufbau mindestens eine Deckschicht aufweist, wobei im Verfahrensschritt b) die Deckschicht derart zu dem Strahlenschirm angeordnet wird, dass die Deckschicht vollständig oder teilweise durch die sterilisierende Strahlung sterilisiert wird.

Ausführungsform 5: Verfahren der vorhergehenden Ausführungsform, wobei die Deckschicht eine Klebeschicht umfasst, insbesondere eine Klebeschicht zum Fixieren des implantierbaren Sensors auf einer Hautoberfläche.

Ausführungsform 6: Verfahren nach einer der beiden vorhergehenden Ausführungsformen, wobei nach Durchführung des Verfahrensschritts b) die Deckschicht innerhalb der Verpackung auf das elektronische Bauelement aufgebracht wird, insbesondere ohne Öffnen der Verpackung.

Ausführungsform 7: Verfahren nach der vorhergehenden Ausführungsform, wobei nach dem Aufbringen der Deckschicht die Deckschicht eine äußere Oberfläche des Elektronikteils bildet.

Ausführungsform 8: Verfahren nach einer der beiden vorhergehenden Ausführungsformen, wobei die Verpackung verformbar ausgestaltet ist, wobei bei einer Verformung der Verpackung der implantierbare Sensor durch mindestens eine Engstelle bewegt wird, insbesondere gezogen wird, wobei durch die Engstelle die Deckschicht auf das elektronische Bauelement aufgepresst wird.

Ausführungsform 9: Verfahren nach der vorhergehenden Ausführungsform, wobei die Engstelle durch mindestens ein Druckkissen in der Verpackung gebildet wird.

Ausführungsform 10: Verfahren nach einer der beiden vorhergehenden Ausführungsformen, wobei die Verpackung einen Faltenbalg umfasst.

Ausführungsform 11: Verfahren nach einem der drei vorhergehenden Ausführungsformen, wobei bei der Verformung der Verpackung der Strahlenschirm von der Verpackung getrennt wird.

Ausführungsform 12: Verfahren nach der vorhergehenden Ausführungsform, wobei der Strahlenschirm vor dem Verformen der Verpackung von außen in mindestens einen in einen Innenraum der Verpackung hineinragenden Finger der Verpackung eingesteckt ist, insbesondere von außen.

Ausführungsform 13: Verfahren nach der vorhergehenden Ausführungsform, wobei der Finger der Verpackung beim Entfernen des Strahlenschirms aus einem Innenraum der Verpackung herausgezogen wird.

Ausführungsform 14: Verfahren nach einer der sechs vorhergehenden Ausführungsformen, wobei die Verpackung derart verformbar ausgestaltet ist, dass der Sensor mit einem ersten Verpackungsteil der Verpackung verbunden ist, wobei der Strahlenschirm mit einem zweiten Verpackungsteil der Verpackung bewegbar ist, wobei während der Verformung der Verpackung der erste Verpackungsteil und der zweite Verpackungsteil derart zueinander verbunden bleiben, dass eine keimdichte Abschirmung des Sensors gewährt ist.

Ausführungsform 15: Verfahren nach einer der sieben vorhergehenden Ausführungsformen, wobei bei der Verformung der Verpackung die Deckschicht großflächig von mindestens einer Seite auf ein das elektronische Bauelement tragendes Substrat des Elektronikteils aufgebracht wird, insbesondere aufgeklebt oder auf laminiert wird.

Ausführungsform 16: Verfahren nach einer der acht vorhergehenden Ausführungsformen, wobei die Verpackung mindestens einen Griff, vorzugsweise mindestens zwei Griffe, zum Halten der Verpackung durch einen die Verformung durchführenden Benutzer aufweist.

Ausführungsform 17: Verfahren nach einer der vorhergehenden Ausführungsformen, wobei der Strahlenschirm mindestens einen metallischen Strahlenschirm umfasst.

Ausführungsform 18: Verfahren nach der vorhergehenden Ausführungsform, wobei der metallische Strahlenschirm eine Dicke von 1 mm bis 10 mm aufweist, vorzugsweise von 3 mm bis 7 mm und besonders bevorzugt von 5 mm.

Ausführungsform 19: Verfahren nach einer der beiden vorhergehenden Ausführungsformen, wobei der metallische Strahlenschirm mindestens ein metallisches Material umfasst, ausgewählt aus der Gruppe bestehend aus: Aluminium, Eisen, Stahl, Blei, Kupfer.

Ausführungsform 20: Verfahren nach einer der vorhergehenden Ausführungsformen, wobei im Verfahrensschritt a) zusätzlich mindestens ein medizinisches Hilfsmittel, insbesondere mindestens eine Insertionshilfe, in die Verpackung eingebracht wird.

Ausführungsform 21: Sterilisationsvorrichtung zum Sterilisieren eines implantierbaren Sensors zur Erfassung mindestens eines Analyten in einem Körpergewebe, insbesondere zum Einsatz in einem Verfahren nach einer der vorhergehenden Ausführungsform, wobei die Sterilisationsvorrichtung umfasst:
- mindestens einen implantierbaren Sensor zur Erfassung mindestens eines Analyten in einem Körpergewebe, wobei der implantierbare Sensor mindestens einen in das Körpergewebe einbringbaren Sensorteil mit mindestens einer Sensorelektrode zur Erfassung des Analyten und mindestens einen Elektronikteil aufweist, wobei der Elektronikteil mindestens ein elektronisches Bauelement aufweist und mit dem Sensorteil verbunden ist;
- mindestens eine Verpackung, wobei die Verpackung den implantierbaren Sensor keimdicht abschließt und wobei die Verpackung mindestens einen das elektronische Bauelement des Elektronikteils während einer Strahlensterilisation abschirmenden Strahlenschirm aufnimmt.

Ausführungsform 22: Sterilisationsvorrichtung nach der vorhergehenden Ausführungsform, wobei die Sterilisationsvorrichtung eingerichtet ist, um in einem Verfahren nach einer oder mehreren der Ausführungsformen 1-19 eingesetzt zu werden.

Ausführungsform 23: Sterilisationsvorrichtung nach einer der beiden vorhergehenden Ausführungsformen, wobei der Sensor mindestens einen ersten Teil und mindestens einen zweiten Teil aufweist, wobei der erste Teil bei der Strahlensterilisation in der Verpackung sterilisierbar ist, wobei der zweite Teil bei der Strahlensterilisation durch den Strahlenschirm abgeschirmt wird, wobei nach Durchführung der Strahlensterilisation der erste Teil und der zweite Teil innerhalb der Verpackung verbindbar sind, insbesondere ohne Öffnen der Verpackung.

Ausführungsform 24: Sterilisationsvorrichtung nach der vorhergehenden Ausführungsform, wobei die Verpackung verformbar ausgestaltet ist, wobei bei einer Verformung der Verpackung nach Durchführung der Strahlensterilisation der erste Teil und der zweite Teil derart zueinander bewegbar sind, dass diese miteinander verbunden werden, insbesondere indem der erste Teil und der zweite Teil gegeneinander gepresst werden.

Ausführungsform 25: Sterilisationsvorrichtung nach einer der vorhergehenden, eine Sterilisationsvorrichtung betreffenden Ausführungsformen, wobei der Elektronikteil einen Schichtaufbau aufweist, wobei der Schichtaufbau mindestens eine Deckschicht aufweist, wobei die Verpackung derart ausgestaltet ist, dass während der Strahlensterilisation die Deckschicht derart zu dem Strahlenschirm angeordnet ist, dass die Deckschicht zumindest teilweise durch die sterilisierende Strahlung sterilisierbar ist, während das elektronische Bauelement abgeschirmt ist.

Ausführungsform 26: Sterilisationsvorrichtung nach der vorhergehenden Ausführungsform, wobei die Verpackung derart ausgestaltet ist, dass nach Durchführung der Strahlensterilisation die Deckschicht innerhalb der Verpackung auf das elektronische Bauelement aufgebracht werden kann, insbesondere ohne Öffnen der Verpackung.

Ausführungsform 27: Sterilisationsvorrichtung nach der vorhergehenden Ausführungsform, wobei die Verpackung verformbar ausgestaltet ist, wobei bei einer Verformung der Verpackung der implantierbare Sensor durch mindestens eine Engstelle bewegt wird, insbesondere gezogen wird, wobei durch die Engstelle die Deckschicht auf das elektronische Bauelement aufpressbar ist.

Ausführungsform 28: Sterilisationsvorrichtung nach der vorhergehenden Ausführungsform, wobei die Engstelle durch mindestens ein Druckkissen in der Verpackung gebildet wird.

Ausführungsform 29: Sterilisationsvorrichtung nach einer der beiden vorhergehenden Ausführungsformen, wobei bei der Verformung der Verpackung der Strahlenschirm von der Verpackung trennbar ist.

Ausführungsform 30: Sterilisationsvorrichtung nach einer der drei vorhergehenden Ausführungsformen, wobei der Strahlenschirm vor dem Verformen der Verpackung von außen in mindestens einen in einen Innenraum der Verpackung hineinragenden Finger der Verpackung eingesteckt ist.

Ausführungsform 31: Sterilisationsvorrichtung nach der vorhergehenden Ausführungsform, wobei der Finger der Verpackung beim Entfernen des Strahlenschirms aus dem Innenraum der Verpackung herausgezogen wird.

Ausführungsform 32: Sterilisationsvorrichtung nach einer der fünf vorhergehenden Ausführungsformen, wobei die Verpackung derart verformbar ausgestaltet ist, dass der Sensor mit einem ersten Verpackungsteil der Verpackung verbunden ist, wobei der Strahlenschirm mit einem zweiten Verpackungsteil der Verpackung bewegbar ist, wobei während der Verformung der Verpackung der erste Verpackungsteil und der zweite Verpackungsteil derart miteinander verbunden bleiben, dass eine keimdichte Abschirmung des implantierbaren Sensors gewährleistet ist.

Ausführungsform 33: Sterilisationsvorrichtung nach einer der vorhergehenden, eine Sterilisationsvorrichtung betreffenden Ausführungsformen, wobei die Verpackung einen Faltenbalg aufweist.

Ausführungsform 34: Sterilisationsvorrichtung nach einer der vorhergehenden, eine Sterilisationsvorrichtung betreffenden Ausführungsformen, wobei die Verpackung mindestens zwei Griffe aufweist.

Ausführungsform 35: Sterilisationsvorrichtung nach einer der vorhergehenden, eine Sterilisationsvorrichtung betreffenden Ausführungsformen, wobei die Verpackung eine Kunststofffolie aufweist.

Ausführungsform 36: Sterilisationsvorrichtung nach einer der vorhergehenden, eine Sterilisationsvorrichtung betreffenden Ausführungsformen, wobei die Verpackung eine Sollbruchstelle zum irreversiblen Öffnen und Entnehmen des implantierbaren Sensors aufweist.

### Kurze Beschreibung der Figuren

Weitere Einzelheiten und Merkmale der Erfindung ergeben sich aus der nachfolgenden Beschreibung von bevorzugten Ausführungsbeispielen, insbesondere in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt. Die Ausführungsbeispiele sind in den Figuren schematisch dargestellt. Gleiche Bezugsziffern in den einzelnen Figuren bezeichnen dabei gleiche oder funktionsgleiche beziehungsweise hinsichtlich ihrer Funktionen einander entsprechende Elemente.

### Im Einzelnen zeigen:

- Figur 1: ein erstes Ausführungsbeispiel einer erfindungsgemäßen Sterilisationsvorrichtung und eines erfindungsgemäßen Verfahrens; und
- Figur 2: ein zweites Ausführungsbeispiel einer erfindungsgemäßen Sterilisationsvorrichtung und eines erfindungsgemäßen Verfahrens;
- Figur 3: eine Detaildarstellung einer Aufnahme eines Strahlenschirms in einer Verpackung;
- Figur 4: ein drittes Ausführungsbeispiel einer erfindungsgemäßen Sterilisationsvorrichtung;
- Figur 5: ein viertes Ausführungsbeispiel einer erfindungsgemäßen Sterilisationsvorrichtung und eines erfindungsgemäßen Verfahrens; und
- Figur 6: ein fünftes Ausführungsbeispiel einer erfindungsgemäßen Sterilisationsvorrichtung und eines erfindungsgemäßen Verfahrens.

### Ausführungsbeispiele

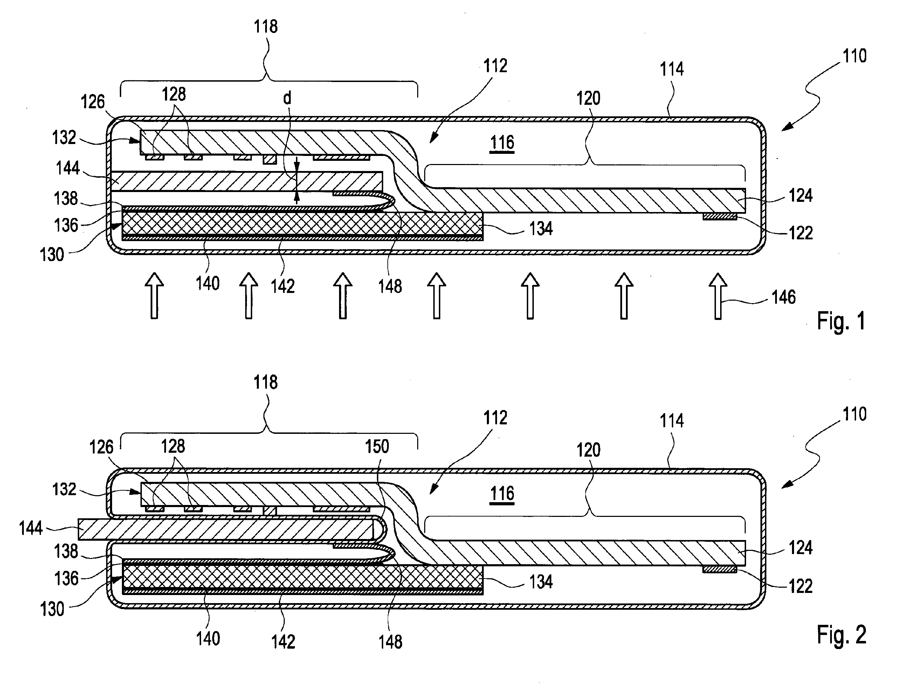

In Figur 1 ist ein erstes Ausführungsbeispiel einer erfindungsgemäßen Sterilisationsvorrichtung 110 zum Sterilisieren eines implantierbaren Sensors 112 in einer schematischen Schnittdarstellung gezeigt. Gleichzeitig zeigt das in Figur 1 dargestellte Ausführungsbeispiel ein Beispiel eines erfindungsgemäßen Verfahrens zum Sterilisieren des implantierbaren Sensors 112.

Die Sterilisationsvorrichtung 110 kann allgemein, wie auch im vorliegenden Ausführungsbeispiel, als Sterilverpackung ausgestaltet sein und weist eine Verpackung 114 auf, welche vorzugsweise keimdicht ausgestaltet ist und welche einen Innenraum 116 aufweist und diesen keimdicht verschließt. In dem Innenraum 116 ist der implantierbare Sensor 112 aufgenommen.

Der implantierbare Sensor 112 weist einen Elektronikteil 118 und einen mit dem Elektronikteil verbundenen, in ein Körpergewebe eines Benutzers implantierbaren Sensorteil 120 auf. Der Sensorteil 120 weist mindestens eine Sensorelektrode 122 auf, in der Regel 2, 3 oder mehr Sensorelektroden 122. Dabei kann es sich insbesondere um enzymatische Sensorelektroden 122 handeln, wie oben beschrieben. Die mindestens eine Sensorelektrode 122 kann beispielsweise auf einem Substrat 124 aufgebracht sein. Beispielsweise kann es sich dabei um ein Kunststoffsubstrat 124 handeln, mit einem einschichtigen oder mehrschichtigen Aufbau. Beispielsweise kann das Substrat 124 auch eine flexible Leiterplatte sein oder umfassen.

Die Sensorelektrode 122 kann über eine oder mehrere in Figur 1 nicht dargestellte Zuleitungen mit dem Elektronikteil 118 verbunden sein. Der Elektronikteil 118 und der Sensorteil 120 können einstückig ausgebildet sein, so dass diese zerstörungsfrei vorzugsweise nicht trennbar sind.

Der Elektronikteil 118 weist vorzugsweise ebenfalls mindestens ein Substrat 126 auf. Beispielsweise kann es sich bei diesem Substrat um einen Schaltungsträger handeln. Vorzugsweise ist dieser Schaltungsträger wiederum flexibel ausgestaltet, beispielsweise in Form einer flexiblen Leiterplatte. Auf und/oder in dem Schaltungsträger und/oder dem Substrat 126 sind ein oder mehrere elektronische Bauelemente 128 angeordnet. Beispielsweise können diese elektronischen Bauelemente 128 ein oder mehrere Halbleiterbauelemente umfassen, insbesondere ein oder mehrere aktive Halbleiterbauelemente wie beispielsweise Verstärkerbauelemente. Insbesondere kann das mindestens eine elektronische Bauelement 128 mindestens einen Potentiostaten umfassen, welcher einstückig oder auch mehrteilig ausgebildet sein kann. Die elektronischen Bauelemente 128 können gemeinsam eine Ansteuer- und/oder Auswerteschaltung des implantierbaren Sensors 112 bilden. Weiterhin können auch ein oder mehrere Schnittstellen für eine Kommunikation des implantierbaren Sensors 112 mit einem oder mehreren weiteren, nicht dargestellten Bauelementen vorgesehen sein.

Der implantierbare Sensor 112 und hier insbesondere der Elektronikteil 118 sind in dem dargestellten Ausführungsbeispiel mehrteilig ausgestaltet und umfassen mindestens einen ersten Teil 130 und mindestens einen zweiten Teil 132. Beispielsweise kann der erste Teil 130 mindestens eine Deckschicht 134 umfassen, welche vorzugsweise später die elektronischen Bauelemente 128 abdeckt. Beispielsweise kann die Deckschicht 134 eine erste Klebeschicht 136 umfassen mittels derer die Deckschicht 134 auf die elektronischen Bauelemente 128 und/oder das Substrat 126 aufgeklebt werden kann. In dem in Figur 1 dargestellten Ausgangszustand kann diese erste Klebeschicht 136 noch mit einer Abdeckschicht 138 bedeckt sein. Diese Abdeckschicht 138 kann später, vor dem Aufkleben der Deckschicht 134 auf das Substrat 126, abgezogen werden. Die Abdeckschicht 138 kann beispielsweise als Schutzfolie ausgestaltet sein.

Weiterhin kann die Deckschicht 134 mindestens eine zweite Klebeschicht 140 aufweisen, auf einer dem Substrat 126 abgewandten Seite. Mittels dieser zweiten Klebeschicht 140, welche allgemein auch durch ein beliebiges Fixierelement ersetzt sein kann, kann beispielsweise der Elektronikteil 118 auf einer Hautoberfläche eines Benutzers des implantierbaren Sensors 112 fixiert werden, während der Sensorteil 120 vollständig oder teilweise durch eine Insertionsöffnung in eine Hautoberfläche eines Benutzers in ein Körpergewebe des Benutzers eingeführt ist oder eingeführt wird. Auch die zweite Klebeschicht 140 kann mit einer Abdeckschicht 142 abgedeckt sein, beispielsweise wiederum mit einer Schutzfolie.

Weiterhin weist die Sterilisationsvorrichtung 110 mindestens einen Strahlenschirm 144 auf. Dieser Strahlenschirm 144 kann beispielsweise, wie in Figur 1 dargestellt, in dem Innenraum 116 aufgenommen sein. Alternativ kann der Strahlenschirm 144 jedoch auch ganz oder teilweise außerhalb des Innenraums 116 aufgenommen sein, als Bestandteil der Verpackung 114 oder als separater Bestandteil.

Der Strahlenschirm 144 kann beispielsweise ein metallischer Strahlenschirm sein, welcher ganz oder teilweise aus mindestens einem metallischen Material, insbesondere aus mindestens einem metallischen Vollmaterial, hergestellt ist. Beispielsweise kann der Strahlenschirm 144 eine Dicke d senkrecht zu einer Bestrahlungsrichtung 146 aufweisen, welche beispielsweise 1 bis 10 mm betragen kann, insbesondere 5 mm. Beispielsweise kann der Strahlenschirm 144 eine Aluminiumplatte umfassen.

Bei einem erfindungsgemäßen Verfahren zum Sterilisieren des implantierbaren Sensors 112 kann zunächst in dem in Figur 1 dargestellten Zustand eine Bestrahlung des Sensors 112 erfolgen, zum Zweck einer Strahlensterilisation, beispielsweise mittels β -Strahlung mit einer Energie von 2,5 MeV. Insbesondere kann der implantierbare Sensor 112 dabei mit einer Strahlendosis von 25 kGy beaufschlagt werden. Auch andere Arten der Strahlensterilisation sind möglich. Die Strahlensterilisation erfolgt vorzugsweise gerichtet, aus einer oder auch, wie unten noch näher beschrieben wird, zwei, drei oder mehr Raumrichtungen. Bei der Strahlensterilisation werden der Sensorteil 120 sowie der erste Teil 130 des Elektronikteils 118, also die Deckschicht 134, durch die Strahlensterilisation sterilisiert. Der Strahlenschirm 144 schirmt hingegen die sterilisierende Strahlung von dem zweiten Teil 132, welcher das mindestens eine elektronische Bauelement 128 oder mindestens eines der elektronischen Bauelemente 128 beinhaltet, ab. Dazu ist der Strahlenschirm 144 vorzugsweise geometrisch derart ausgelegt, dass bei allen in Frage kommenden Raumrichtungen der sterilisierenden Strahlung eine zuverlässige Abschirmung des zweiten Teils 132, welches das mindestens eine elektronische Bauelement 128 oder mindestens eines der elektronischen Bauelemente 128 beinhaltet, sicher gestellt ist. Insbesondere können auf diese Weise sämtliche elektronische Bauelemente 128 oder zumindest ein oder mehrere dieser elektronischen Bauelemente 128, beispielsweise mindestens ein empfindliches Bauelement der elektronischen Bauelemente 128, vor der sterilisierenden Strahlung abgeschirmt werden.

Die Verpackung 114 kann beispielsweise ganz oder teilweise als Kunststoffverpackung ausgestaltet sein. Insbesondere kann die Verpackung 114 eine Kunststofffolie umfassen. Nach der Strahlensterilisation kann die Verpackung 114 derart gehandhabt werden, dass innerhalb der geschlossenen Verpackung 114, ohne dass ein Öffnen der Verpackung 114 erforderlich wäre, der erste Teil 130 und der zweite Teil 132 verbunden werden. Insbesondere kann dabei die Deckschicht 134 auf das mindestens eine elektronische Bauelement 128 und/oder das Substrat 126 aufgebracht werden, beispielsweise auf laminiert und/oder aufgeklebt werden. Um dies zu bewerkstelligen, kann die Verpackung 114 beispielsweise flexibel ausgestaltet werden. Dabei kann die Verpackung 114 beispielsweise derart verformt werden, dass der Strahlenschirm 144 aus dem Bereich zwischen dem ersten Teil 130 und dem zweiten Teil 132 entfernt wird und der zweite Teil 132 auf den ersten Teil 130 aufgebracht wird. Beispielsweise kann ein Bedienpersonal unmittelbar nach dem Sterilisationsvorgang das linke Ende der Verpackung 114 in Figur 1 sowie das rechte Ende der Verpackung 114 ergreifen und auseinanderziehen. Vorzugsweise ist in dieser Ausgestaltung der Strahlenschirm 144 mit dem linken Ende der Verpackung 114 verbunden, so dass bei dem Auseinanderziehen eine Verformung der Verpackung 114 derart erfolgt, dass der Strahlenschirm 144 aus dem Bereich zwischen dem ersten Teil 130 und dem zweiten Teil 132 herausgezogen wird. Der Strahlenschirm 144 kann, wie in Figur 1 ebenfalls als Option dargestellt, über mindestens eine Verbindung 148 mit der Abdeckschicht 138, beispielsweise der Schutzfolie der Klebeschicht 136, verbunden sein. Dementsprechend kann beim Herausziehen des Strahlenschirms 144 aus dem Zwischenraum zwischen dem ersten Teil 130 und dem zweiten Teil 132 auch gleichzeitig die Abdeckschicht 138 entfernt werden. Auch andere Ausgestaltungen sind jedoch grundsätzlich möglich, beispielsweise ein separates Entfernen der Abdeckschicht 138 und/oder einer Ausgestaltung ohne Abdeckschicht 138 und/oder ohne erste Klebeschicht 136.

Gleichzeitig kann durch den oben beschriebenen Verformungsvorgang beim Auseinanderziehen der Verpackung 114 die Höhe der Verpackung 114 in Figur 1 derart reduziert werden, dass der erste Teil 130 und der zweite Teil 132 zusammengepresst werden. Auf diese Weise kann aus dem in Figur 1 dargestellten, als Halbfertigteil ausgestalteten implantierbaren Sensor 112 ein fertiger, verwendbarer implantierbarer Sensor 112 entstehen, bei welchem die Deckschicht 134 auf den Elektronikteil 118 aufgebracht ist. Sämtliche, dem Benutzer zuweisenden Oberflächen des implantierbaren Sensors 112 sind somit sterilisiert, ohne dass die elektronischen Bauelemente 128 geschädigt werden.

Die derart vorbereitete Sterilisationsvorrichtung 110 gemäß Figur 1 kann dann beispielsweise an einen Benutzer ausgeliefert werden. Alternativ kann das Auseinanderziehen der Verpackung 114 auch bei einem Benutzer erfolgen. Ein Benutzer kann dann beispielsweise die Verpackung 114 öffnen und den implantierbaren Sensor 112 für eine Verwendung übernehmen. Dabei kann, beispielsweise mit einer in Figur 1 nicht dargestellten Insertionshilfe, welche ebenfalls im Innenraum 116 aufgenommen sein kann, oder welche als separates Bauteil ausgestaltet sein kann, der Sensorteil 120 vollständig oder teilweise in ein Körpergewebe insertiert werden. Der Elektronikteil 118 kann vollständig oder teilweise mittels der zweiten Klebeschicht 140 auf eine Hautoberfläche des Benutzers fixiert werden, indem beispielsweise die Abdeckschicht 142 abgezogen und die zweite Klebeschicht 140 auf die Hautoberfläche aufgepresst wird.

Wie oben ausgeführt, kann während der Verformung der Verpackung 114, welcher beispielsweise als Kunststofffolie und insbesondere als PE-Folie (PE: Polyethylen) ausgestaltet sein kann, die Fertigmontage des implantierbaren Sensors 112 erfolgen. Vorzugsweise kann die Verpackung 114 bei dieser Verformung geschlossen bleiben, so dass der Innenraum 116 durch die Verpackung 114 noch vor einer Wiederverkeimung geschützt wird. Alternativ kann bei der Verformung der Verpackung 114 die Verpackung 114 jedoch auch aufgerissen werden, so dass beispielsweise das Entfernen des Strahlenschirms 144 und die Fertigmontage des implantierbaren Sensors 112 bei einem Öffnen der Verpackung 114 erfolgen können.

Die Klebeschichten 136, 140 können beispielsweise als beidseitige Klebefilme ausgestaltet sein. Die der Hautoberfläche zuweisende zweite Klebeschicht 140 kann beispielsweise als flexibles, atmungsaktives Pflaster ausgestaltet sein.

Der Strahlenschirm 144 kann bevorzugt aus Aluminium gefertigt sein. Aluminium stellt einen guten Kompromiss bezüglich einer Absorption von Elektronen und einer geringen Bremsstrahlung dar. Es ist allerdings auch möglich, andere Materialien zu verwenden, beispielsweise metallische Materialien. Bevorzugt kann ein Verbund aus einem Leichtmetall und einem Schwermetall eingesetzt werden, besonders bevorzugt ein Verbund aus Aluminium und Blei, wobei beispielsweise die Aluminiumseite der Strahlenquelle zugewandt ist.

Der Elektronikteil 118 ist in Figur 1 lediglich symbolisch dargestellt. Insbesondere kann das mindestens eine elektronische Bauelement 128 auch auf andere Weise als in Figur 1 dargestellt angeordnet und/der ausgestaltet sein. So können auch ein oder mehrere elektronische Bauelemente 128, alternativ oder zusätzlich zu der in Figur 1 dargestellten Anordnung auf der der Bestrahlungsrichtung 146 zugewandten Seite, in das Substrat 126 integriert und/oder auf einer der Bestrahlungsrichtung 146 abgewandten, gegenüberliegenden Seite des Substrats 126 angeordnet sein. Wiederum alternativ oder zusätzlich kann das mindestens eine elektronische Bauelement 128 vollständig oder teilweise auch in ein Substratmaterial eingebettet sein, beispielsweise mit einem Kunstharz vergossen sein, so dass eine ebene Oberfläche entsteht, die gut mit der Deckschicht 134 und insbesondere der ersten Klebeschicht 136 verklebt oder auf andere Weise verbunden werden kann.

Grundsätzlich wäre es auch möglich, den Strahlenschirm 144 vor der Sterilisation mit der Klebeschicht und dem sich darüber befindlichen Substrat 126 sowie dem mindestens einen elektronischen Bauelement 128 zu verbinden, beispielsweise zu vergießen und/oder permanent zu verkleben. Eine derartige Ausgestaltung hätte jedoch grundsätzlich ein unerwünscht hohes Profil des Sensorsystems zur Folge, da bei dieser Ausgestaltung bei typischen Dicken des Strahlenschirms 144 von 5 mm eine hohe Bauhöhe des Elektronikteils 118 entstehen würde.

In Figur 2 ist eine alternative Ausgestaltung der Sterilisationsvorrichtung 110 in einer zu Figur 1 analogen Darstellung gezeigt. Dementsprechend kann zunächst für die dargestellten Bauelemente weitgehend auf die Beschreibung der Figur 1 verwiesen werden.

ln Figur 2 ist jedoch eine Alternative dargestellt, bei welcher der Strahlenschirm 144 nicht im Innenraum 116 aufgenommen ist, sondern außerhalb des Innenraums 116. Der Strahlenschirm 144 ist somit vorzugsweise nicht Bestandteil der Verpackung 114 sondern separat von dieser Verpackung 114 ausgebildet. Beispielsweise kann der Strahlenschirm 144 allgemein bei diesem Ausführungsbeispiel oder in anderen Ausführungsbeispielen jedoch derart in den Innenraum 116 hineinragen, dass dieser vor der Endmontage des implantierbaren Sensors 112 in einem Zwischenraum zwischen dem ersten Teil 130 und dem zweiten Teil 132 angeordnet ist. Auf diese Weise kann der Strahlenschirm 144 angeordnet sein, um aus einer oder mehreren Raumrichtungen auftreffende, in Figur nicht dargestellte sterilisierende Strahlung von dem mindestens einem elektronischen Bauelement 128 abzuschirmen, während der erste Teil 130, beispielsweise die Deckschicht 134, durch die sterilisierende Strahlung sterilisiert wird.

Beispielsweise kann der Strahlenschirm 144 an mindestens einem Vorsprung ins Innere des Innenraums 116 hineinragen. Beispielsweise kann die Verpackung 114 mindestens einen in den Innenraum 116 hineinragenden Finger 150 in Form einer Tasche aufweisen, in welche von außen der Strahlenschirm 144 eingesteckt und/oder eingelegt ist und/oder in welche der Strahlenschirm 144 hineinragt. Der Strahlenschirm 144 kann nach der Strahlensterilisation leicht aus dem Finger 150, insbesondere der Tasche, herausgezogen werden. Beispielsweise kann der Strahlenschirm 144 fest mit einer Fertigungseinrichtung verbunden sein. Bei einer Montage der Sterilisationsvorrichtung 110 gemäß Figur 2 kann beispielsweise die Verpackung 114 mit einer erfindungsgemäßen Tasche bzw. einem erfindungsgemäßen Finger 150 auf diesen Strahlenschirm 144 aufgeschoben und nach der Sterilisation wieder abgezogen werden. Dies hat den Vorteil, dass der Strahlenschirm 144 immer wieder verwendet werden kann.

Wie oben ausgeführt, kann bei dem Herausziehen des Strahlenschirms 144 beispielsweise auch die mindestens eine Abdeckschicht 138 von der ersten Klebeschicht 136 abgezogen werden, beispielsweise indem eine Schutzfolie abgezogen wird. Zu diesem Zweck kann beispielsweise wiederum eine Verbindung 148 vorgesehen sein, welche in diesem Fall die Abdeckschicht 138 vorzugsweise nicht unmittelbar mit dem Strahlenschirm 144 verbindet sondern mit dem Finger 150, insbesondere der Tasche, in welche der Strahlenschirm 144 eingesteckt ist. Der Finger 150 kann derart ausgeführt sein, dass dieser beim Herausziehen des Strahlenschirms 144 mitgenommen und nach außen umgestülpt wird. Dabei wird dann auch über die Verbindung 148 die Abdeckschicht 138, insbesondere die Schutzfolie, abgezogen, und eine Klebeverbindung zwischen dem ersten Teil 130 und dem zweiten Teil 132 wird ermöglicht.

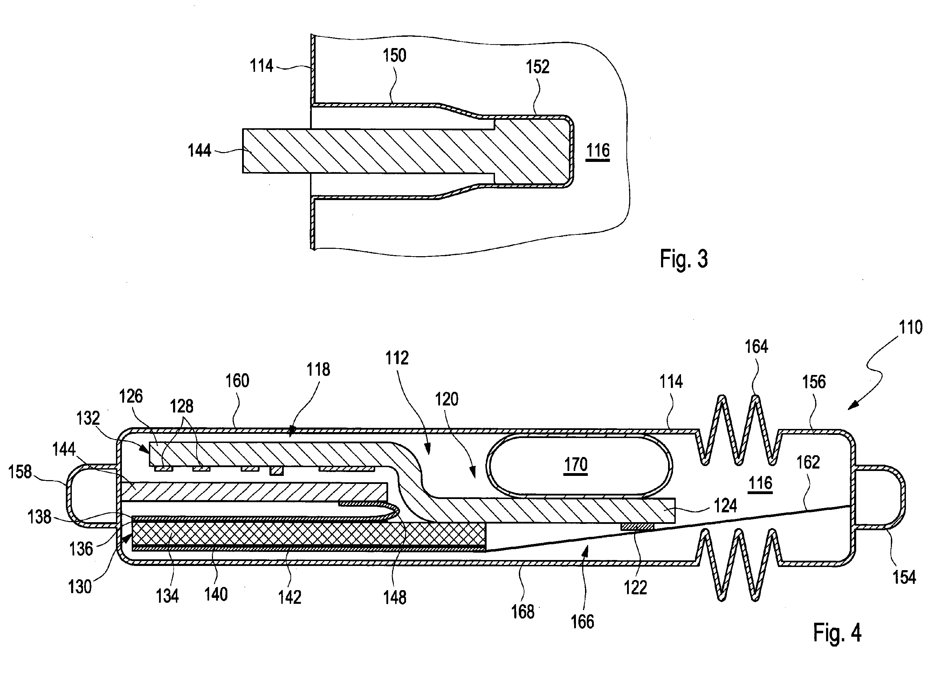

Das Herausziehen des Fingers 150 beim Entfernen des Strahlenschirms 144 kann durch einen einfachen Klemmbereich ermöglicht werden, wie in Figur 3 schematisch dargestellt. So zeigt Figur 3 eine optionale Ausgestaltung des Bereichs des Fingers 150 der Verpackung 114. Das Ausführungsbeispiel zeigt, dass der Finger 150, welcher insbesondere als in den Innenraum 116 hineinragende Tasche ausgestaltet sein kann, insbesondere einen Klemmbereich 152 aufweisen kann, an welchem die Verpackung 114 eng an dem Strahlenschirm 144 anliegt. Wird der Strahlenschirm 144 aus dem Finger 150 herausgezogen, in Figur 3 mit Zugrichtung nach links, und/oder wird die Verpackung 114 von dem Strahlenschirm 144 abgezogen, in Figur 3 mit Bewegungsrichtung nach rechts, so kann die Wand der Verpackung 114 im Bereich des Klemmbereichs 152 an dem Strahlenschirm 144 festsitzen, so dass der Finger 150 aus dem Innenraum 116 herausgezogen wird.

In Figur 4 ist ein drittes Ausführungsbeispiel einer erfindungsgemäßen Sterilisationsvorrichtung 110 dargestellt. Die Sterilisationsvorrichtung 110 kann zunächst wiederum weitgehend dem Ausführungsbeispiel gemäß Figur 1 entsprechen, so dass bezüglich der meisten Elemente dieses Ausführungsbeispiels auf die obige Beschreibung der Figur 1 verwiesen werden kann. Wiederum sind der erste Teil 130 und der zweite Teil 132 des in der Verpackung 114 aufgenommenen implantierbaren Sensors 112 in dem in Figur 4 dargestellten Zustand, welcher vor und/oder während der Strahlensterilisation vorliegt, durch den Strahlenschirm 144 getrennt. Die Bestrahlung kann wieder, analog zu Figur 1, in Figur 4 von unten erfolgen, so dass der erste Teil 130 strahlensterilisiert wird, der zweite Teil 132 jedoch durch den Strahlenschirm 144 vollständig oder teilweise abgeschirmt wird.

Das Ausführungsbeispiel gemäß Figur 4 weist gegenüber dem Ausführungsbeispiel in Figur 1 eine Mehrzahl von Modifikationen auf, welche einzeln oder in beliebiger Kombination realisierbar sind.

So zeigt das Ausführungsbeispiel gemäß Figur 4 zunächst, dass die Verpackung 114 einen oder mehrere Griffe aufweisen kann. So ist in dem Ausführungsbeispiel gemäß Figur 4 ein erster Griff 154 vorgesehen, welcher am rechten Ende der Verpackung 114 angeordnet ist und welcher mit einem ersten Verpackungsteil 156 der Verpackung 114 verbunden ist. An einem in Figur 4 linken Ende der Verpackung 114 ist ein weiterer Griff 158 vorgesehen, welcher mit einem zweiten Verpackungsteil 160 der Verpackung 114 verbunden ist. Mittels der Griffe 154, 158 können die Verpackungsteile 156, 160 auseinandergezogen werden, vorzugsweise ohne dass die Verpackung 114 dabei geöffnet wird. Der implantierbare Sensor 112 ist vorzugsweise mit dem ersten Verpackungsteil 156 verbunden, beispielsweise, wie in Figur 4 exemplarisch dargestellt, über ein Zugband 162. Dieses Zugband kann grundsätzlich an einem beliebigen Teil des implantierbaren Sensors 112 angreifen, beispielsweise, wie in Figur 4 gezeigt, an der Deckschicht 134. Auch andere Ausgestaltungen sind möglich.

Während der Strahlensterilisation wird der implantierbare Sensor 112 in der Sterilisationsvorrichtung 110 gemäß der in Figur 4 gezeigten Konfiguration strahlensterilisiert, beispielsweise mit der in Figur 1 gezeigten Bestrahlungsrichtung 146. Nach der Strahlensterilisation kann, durch Ergreifen der Verpackung 114 an den Griffen 154, 158 und ein Auseinanderziehen der Verpackungsteile 156, 160, die Verpackung 114 gestreckt werden. Dabei wird beispielsweise der Strahlenschirm 144 aus dem Zwischenraum zwischen dem ersten Teil 130 und dem zweiten Teil 132 herausgezogen. Um ein Rei-βen der Verpackung 114 zu verhindern, können der erste Verpackungsteil 156 und der zweite Verpackungsteil 160 durch einen Faltenbalg 164 verbunden sein. Alternativ oder zusätzlich ist jedoch auch die Verwendung einer Verpackung 114 aus einem flexiblen Verpackungsmaterial denkbar, beispielsweise einer flexiblen Folie.

Figur 4 zeigt weiterhin ein Ausführungsbeispiel, bei welchem während der Verformung der Verpackung 114 durch das Auseinanderziehen der Griffe 154, 158 der Sensor endmontiert wird, indem der erste Teil 130 und der zweite Teil 132 miteinander verbunden werden. Zum Zweck dieser Endmontage können der implantierbare Sensor 112 oder Teile desselben durch eine Engstelle 166 in der Verpackung 114 gezogen werden. Diese Option ist in Figur 4 gezeigt. Die Engstelle wird in diesem Ausführungsbeispiel exemplarisch durch einen Zwischenraum zwischen einer Wand 168 der Verpackung 114 und einem Druckkissen 170 gebildet. Auch andere Ausgestaltungen sind denkbar, beispielsweise Ausgestaltungen mit zwei oder mehren Druckkissen 170 und/oder Ausgestaltungen, bei welchen die Engstelle 166, beispielsweise einen Spalt, durch andere Elemente der Verpackung 114 gebildet wird. In der Engstelle 166 können der erste Teil 130 und der zweite Teil 132 aufeinander gepresst werden, so dass beispielsweise die Deckschicht 134, insbesondere mittels der ersten Klebeschicht 136, auf das Substrat 126 des Elektronikteils 118 und/oder auf das elektronische Bauelement 128 gepresst wird. Auf diese Weise kann eine Festigkeit der Verbindung des dadurch entstehenden Schichtaufbaus deutlich erhöht werden.

Mittels der in Figur 4 gezeigten Ausgestaltung kann insbesondere die steril dichte Umhüllung bei der Fertigmontage des implantierbaren Sensors 112 erhalten bleiben. Das Anbringen der Griffe 154, 158 erlaubt ein bequemes Strecken der Verpackung 114. Anstelle des Faltenbalgs 164, der zur Expansion der Verpackung 114 dient, kann, wie oben ausgeführt, auch eine flexible Verpackung oder eine andere Art einer expandierbaren Verpackung verwendet werden. Bevorzugt bleibt jedoch bei sämtlichen Ausgestaltungen dieser Art von Verpackungen 114 die Anforderung einer Sterildichtigkeit während der Verformung der Verpackung 114 und der Fertigmontage des implantierbaren Sensors 112 erhalten.

Durch das in die Verpackung 114 integrierte Druckkissen 170 ist es möglich, schonend Druck auf die Komponenten des implantierbaren Sensors 112 auszuüben, beispielsweise auf das Substrat 126 und insbesondere auf die flexible Leiterplatte. Insbesondere kann auf diese Weise die flexible Leiterplatte vollständig mit der ersten Klebeschicht 136 kontaktiert und verbunden werden. Alternativ oder zusätzlich zu dem Druckkissen 170 können auch andere flexible und/oder starre und/oder halbstarre Bauteile eingesetzt werden.

Zur Gewährleistung einer Relativbewegung zwischen der Verpackung 114 und dem implantierbaren Sensor 112 ist vorzugsweise das Zugband 162 vorgesehen. Dieses Zugband 162 kann sich beispielsweise zwischen dem Griff 154 rechts und der Deckschicht 134 erstrecken. Es sind auch andere Kontaktmöglichkeiten eines solchen Zugbandes 162 möglich. Vorzugsweise sollte dieses Zugband 162 leicht zu entfernen sein. Beispielsweise kann das Zugband 162 daher an der Abdeckschicht 142 der zweiten Klebeschicht 140 angreifen, beispielsweise an einer abziehbaren Schutzfolie. Auch andere Ausgestaltungen sind möglich. Alternativ oder zusätzlich zur Verwendung eines Zugbandes 162 kann auch eine andere Art der Verbindung zwischen der Verpackung 114, insbesondere dem ersten Verpackungsteil 156, und dem implantierbaren Sensor 112 erfolgen. So kann beispielsweise auch eine starre oder teilweise starre Verpackung 114 gewählt werden, welche mechanisch mit dem implantierbaren Sensor 112 interagieren kann und bei der ein expandierbarer Bereich, beispielsweise in Form des Faltenbalgs 164, zwischen einer Ankopplung der Verpackung 114 an den implantierbaren Sensor 112 und einer Anbindung des Strahlenschirms 144 an die Verpackung 114 positioniert ist.

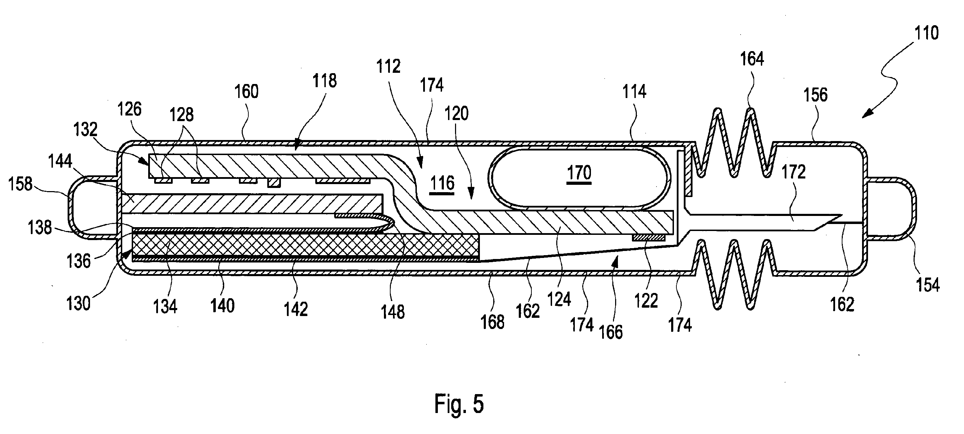

Zusammen mit dem Abziehen des Strahlenschirms 144 und der Freilegung der ersten Klebeschicht 136 können innerhalb der Verpackung 114 auch ein oder mehrere weitere Montageschritte vollzogen werden, bei denen der implantierbare Sensor 112 und/oder ein den implantierbaren Sensor 112 umfassendes Sensorsystem fertig montiert oder weiter montiert werden. Eine derartige Ausgestaltung ist exemplarisch in Figur 5 gezeigt. Die dort dargestellte Sterilisationsvorrichtung 110 kann zunächst wiederum exemplarisch der Ausgestaltung gemäß Figur 4 entsprechen, so dass auf die obige Beschreibung der Figur 4 verwiesen werden kann. Grundsätzlich sind jedoch auch andere Ausgestaltungen möglich.

Zusätzlich zu dem mindestens einen implantierbaren Sensor 112 enthält der Innenraum 116 der Verpackung 114 in dem dort dargestellten Ausführungsbeispiel weiterhin mindestens ein medizinisches Hilfsmittel, in diesem Ausführungsbeispiel mindestens eine Insertionshilfe 172. Alternativ oder zusätzlich zu der Insertionshilfe 172 können ein oder mehrere auf andere Weise gestaltete medizinische Hilfsmittel in der Verpackung 114 aufgenommen sein. Die Insertionshilfe 172 kann beispielsweise als Schlitzkanüle oder auf andere Weise ausgestaltet sein. So ist es in der Regel nötig, die Spitze des implantierbaren Sensors 112 mit dem Sensorteil 120 vollständig oder teilweise subkutan zu implantieren. Als Insertionshilfe 172 kann, wie oben ausgeführt, eine Kanüle, insbesondere eine so genannte Schlitzkanüle mit einem sich entlang der Längsachse der Kanüle erstreckenden Schlitz eingesetzt werden. Die Insertionshilfe 172, insbesondere die Schlitzkanüle, muss dabei in der Regel ebenso sterilisiert werden, wie die Spitze des implantierbaren Sensors 112. Es ist daher in der Regel sinnvoll, die Strahlensterilisation gemeinsam vorzunehmen und die Zusammenführung der Komponenten innerhalb der geschlossenen Verpackung 114 durchzuführen. Dieser mindestens eine Montageschritt kann durch eine entsprechende Ausführung der Verpackung 114 vom Endverbraucher selbst durchgeführt werden. Alternativ kann diese Montage jedoch auch ganz oder teilweise bei einem Hersteller durchgeführt werden, da es in vielen Fällen sinnvoll sein kann, einen derart kritischen Montageschritt bei einer Fertigung im Werk durchzuführen. Die Verpackung 114 kann bei dem in Figur 5 dargestellten Ausführungsbeispiel oder auch bei allen anderen Ausführungsbeispielen der vorliegenden Erfindung insbesondere vollständig oder teilweise transparent ausgestaltet sein. Eine derartige transparente Ausgestaltung der Verpackung 114 bietet auch die Chance, eine optische Kontrolle des Montagevorgangs durchzuführen und dadurch sicher zu stellen, dass eventuelle Fehler entdeckt und behoben werden.

Wie oben ausgeführt, kann die Verpackung 114 insbesondere vollständig oder teilweise aus mindestens einem Folienelement hergestellt sein. Beispielsweise können zu diesem Zweck Kunststofffolien eingesetzt werden, insbesondere flexible oder dehnbare Kunststofffolien wie beispielsweise PE-Folien und/oder PET-Folien. Alternativ oder zusätzlich können für die Verpackung 114 auch starre Materialien verwendet werden. Beispielsweise können Bestandteile der Verpackung 114 als Spritzgießteil und/oder als Spritzblasteil ausgestaltet sein oder auf andere Weise als Kunststoffformteil. Die derartig hergestellten Formteile können beispielsweise mindestens eine Öffnung aufweisen, durch welche der Sensor 112 in den Innenraum 116 eingebracht werden kann. Diese mindestens eine Öffnung kann beispielsweise durch eine Folie verschlossen werden und/oder mit einem Folienelement verbunden werden und/oder mittels eines flexiblen Elements, beispielsweise des Faltenbalgs 164, mittels eines weiteren Verpackungsteils wie beispielsweise einem Stopfen oder eines Schraubverschlusses verschlossen werden. Beispielsweise können die Verpackungsteile 156, 160 ganz oder teilweise als Kunststoffformteile ausgestaltet sein, welche über den Faltenbalg 164 und/oder auf eine andere Weise gestaltetes flexibles Element verbunden werden.

Beispielsweise kann es vorteilhaft sein, bei der Ausgestaltung gemäß Figur 1 die Verpackung 114 vollständig als Folienelement auszugestalten. Bei anderen Ausgestaltungen, wie beispielsweise in den Figuren 2 bis 5 kann es vorteilhaft sein, die Verpackung 114 als starres oder halbstarres Gebilde, beispielsweise als Kunststoff, insbesondere als Kunststoffformteil, herzustellen, beispielsweise in einem Spritzgussverfahren oder Spritzblasverfahren. Eine solche starre Verpackung kann eine große Öffnung aufweisen. Durch diese Öffnung kann der implantierbare Sensor 112 eingelegt werden. Die Öffnung kann mit einer geeigneten Folie verschlossen werden, die nach der Sterilisation und Fertigmontage durch Abziehen die Öffnung wieder freigibt und damit die Entnahme des sterilen implantierbaren Sensors 112 ermöglicht.

Die Ausführung der Verpackung 114 vollständig oder teilweise als Kunststoffformteil ermöglicht in der Regel auch eine präzise Positionierung von Komponenten und die fehlerfreie Zusammenführung des Sensorteils 120 des implantierbaren Sensors 112 und der Insertionshilfe 172, beispielsweise der Schlitzkanüle. Die Verpackung 114 kann auch so ausgeführt werden, dass in einem ersten Schritt die oben beschriebenen Montagen bzw. Positionierungen erfolgen, bei welcher die mindestens zwei Teile 130, 132 zusammengeführt und montiert werden und/oder bei welcher der implantierbare Sensor 112 mit der Insertionshilfe 172 verbunden wird und in einem weiteren Schritt die Öffnung zur Vorbereitung der Entnahme des Sensorsystems mit dem mindestens einen implantierbaren Sensor 112 sowie ggf. der Insertionshilfe 172 erfolgt. Die beiden Schritte können auch ineinander übergehen und innerhalb einer einzigen Bewegung erfolgen. Beispielsweise kann die Verpackung 114, wie oben ausgeführt, mindestens eine Sollbruchstelle umfassen. In Figur 5 sind exemplarisch mögliche Positionen der mindestens einen Sollbruchstelle mit der Bezugsziffer 174 bezeichnet. Alternativ oder zusätzlich sind auch andere Positionen dieser Sollbruchstelle 174 möglich.

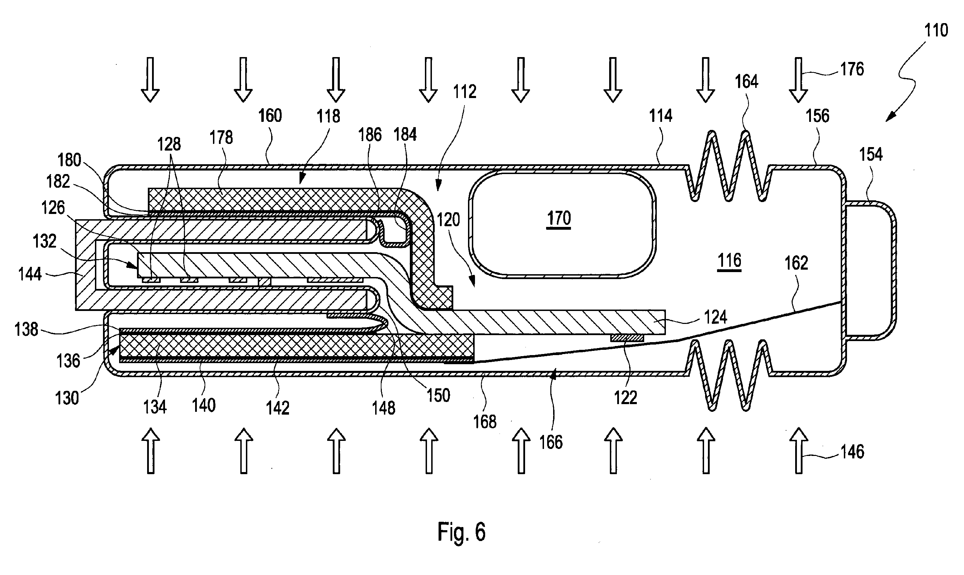

Die Strahlensterilisation kann allgemein, wie in Figur 1 angedeutet, aus einer Bestrahlungsrichtung 146 erfolgen. Dies ist jedoch nicht notwendigerweise der Fall, da grundsätzlich auch mehrere Bestrahlungsrichtungen verwendet werden können. Dies ist exemplarisch in Figur 6 dargestellt. Das Ausführungsbeispiel in Figur 6 stellt zunächst eine Kombination der Ausführungsbeispiele in den Figuren 2 und 4 dar, so dass auf die obige Beschreibung dieser Figuren 2 und 4 verwiesen werden kann. So kann die Verpackung 114 beispielsweise wiederum ein Druckkissen 170 umfassen, sowie mindestens einen verformbaren Bereich, insbesondere einen Faltenbalg 164. Weiterhin kann die Verpackung 114 mindestens einen Griff 154 umfassen, in diesem Fall exemplarisch an einem ersten Verpackungsteil 156. Auch andere Ausgestaltungen sind möglich.

Analog zu dem Ausführungsbeispiel gemäß Figur 2 ist der implantierbare Sensor 112 in Figur 6 wiederum in einen ersten Teil 130 und einen zweiten Teil 132 unterteilt, insbesondere im Bereich des Elektronikteils 118, wobei die Teile 130, 132 auf unterschiedliche Weise strahlensterilisiert werden. Während der erste Teil 132, der das Substrat 126 und ein oder mehrere der elektronischen Bauelemente 128 umfasst, durch einen Strahlenschirm 144 abgeschirmt wird, wird der zweite Teil 132, welcher in diesem Fall mehrteilig ausgestaltet ist, strahlensterilisiert. Im Unterschied zu dem Ausführungsbeispiel gemäß Figur 2, bei welchem vorzugsweise lediglich aus einer Bestrahlungsrichtung (in Figur 2 nicht dargestellt, analog zu Figur 1) die Bestrahlung erfolgt, erfolgt in Figur 6 jedoch eine beidseitige Bestrahlung, aus der Bestrahlungsrichtung 146 sowie einer weiteren Bestrahlungsrichtung 176. In diesem Ausführungsbeispiel kann der implantierbare Sensor 112 somit beidseitig strahlensterilisiert werden. Der Strahlenschirm 144 kann dabei derart ausgestaltet sein, dass dieser den zweiten Teil 132 aus mindestens zwei Raumrichtungen abschirmt, beispielsweise indem der Strahlenschirm 144, wie in Figur 6 gezeigt, den zweiten Teil 132 umschließt. Beispielsweise kann der Strahlenschirm 144 U-förmig ausgestattet sein, wie in Figur 6 angedeutet, wobei zwischen den Schenkeln des U der zweite Teil 132 des implantierbaren Sensors 112 während der Strahlensterilisation angeordnet ist.

Dabei kann, analog zu den Ausgestaltungen in den Figuren 1, 4 und 5, der Strahlenschirm 144 wiederum vollständig oder teilweise in der Verpackung 114 angeordnet sein. Alternativ oder zusätzlich kann der Strahlenschirm 144 jedoch auch, wie in Figur 6 gezeigt, analog zu Figur 2 lediglich in die Verpackung 114 hineinragen und von der Verpackung 114 trennbar ausgestaltet sein. Beispielsweise können zu diesem Zweck wiederum ein oder mehrere Finger 150, 186 vorgesehen sein, welche ins Innere der Verpackung 114 ragen und in welcher der Strahlenschirm 144 eingesteckt ist. Diese Finger 150, 186 können allgemein wiederum als Taschen ausgebildet sein, analog zu Figur 2.

So weist der erste Teil 130 wiederum, wie in Figur 6 gezeigt, eine Deckschicht 134 auf, mit einer ersten Klebeschicht 136, die durch eine Abdeckschicht 138 abgedeckt ist. Wird nach der Strahlensterilisation der zwischen der Deckschicht 134 und dem zweiten Teil 132 angeordneten Finger 150 der Verpackung 114 aus dem Innenraum 116 herausgezogen, so kann über eine Verbindung 148 die Abdeckschicht 138 abgezogen werden, und die Deckschicht 134 kann auf das mindestens eine elektronische Bauelement 128 und/oder auf das Substrat 126 aufgeklebt werden.

Weiterhin kann auch von einer der weiteren Bestrahlungsrichtung 176 zuweisenden Seite eine weiter Deckschicht 178 auf das Substrat 126 aufgebracht werden, beispielsweise auf die flexible Leiterplatte. Diese weitere Deckschicht 178 kann aus der Bestrahlungsrichtung 176 strahlensterilisiert werden, beispielsweise in zur Bestrahlungsrichtung 146 analoger Weise, beispielsweise wiederum mit β -Strahlung mit einer Energie von 2,5 MeV. Die weitere Deckschicht 178 kann beispielsweise wiederum mit einer weiteren Klebeschicht 180 sowie einer weiteren Abdeckschicht 182 versehen sein. Auf einer der weiteren Deckschicht 178 zuweisenden Seite kann die Verpackung 114 mindestens einen weiteren Finger 186 aufweisen, in welchen in der in Figur 6 gezeigten Konfiguration der weiteren Deckschicht 178 zuweisende Schenkel des Strahlenschirms 144 hineinragt. Dieser weitere Finger 186 kann über eine weitere Verbindung 184 mit der weiteren Abdeckschicht 182 verbunden sein, so dass beim Herausziehen des Strahlenschirms 144 und beim Herausziehen der Finger 150, 186 über die Verbindungen 148, 184 die Abdeckschichten 138, 182 abgezogen werden.

Bei der Strahlensterilisation werden die Deckschichten 134 und 178 strahlensterilisiert, wohingegen das Substrat 126 und das mindestens eine elektronische Bauelement 128 durch den Strahlenschirm 144 vollständig oder teilweise abgeschirmt werden. Nach der Strahlensterilisation kann, bei einem Hersteller und/oder bei einem Benutzer des implantierbaren Sensors 112, anschließend eine Fertigmontage des implantierbaren Sensors 112 erfolgen, indem die Verpackung 114 verformt wird. Dabei kann optional der Strahlenschirm 144 aus der Verpackung 114 herausgezogen werden. Alternativ oder zusätzlich kann der Strahlenschirm 144 jedoch auch, analog zu den Ausgestaltungen in den Figuren 1, 4 und 5, vollständig oder teilweise in der Verpackung 114 verbleiben. Weiterhin kann bei der Verformung der Verpackung 114 die Fertigmontage des implantierbaren Sensors 112 erfolgen, beispielsweise analog zu den oben beschriebenen Ausführungsbeispielen, wobei optional zusätzlich noch eine Montage mit einer in Figur 6 nicht dargestellten Insertionshilfe 172 erfolgen kann, analog beispielsweise zu Figur 5. Wiederum kann bei der Fertigmontage bei der Verformung der Verpackung 114 beispielsweise der implantierbare Sensor 112 durch mindestens eine Engstelle 166 in der Verpackung 114 gezogen werden, wie in Figur 6 angedeutet. Diese kann beispielsweise wiederum durch ein Druckkissen 170 gebildet werden. Auch andere der oben beschriebenen Ausgestaltungen der Engestelle sind möglich. Beispielsweise kann zu diesem Zweck wiederum eine Verbindung zwischen dem implantierbaren Sensor 112 und einem ersten Verpackungsteil 156 erfolgen, beispielsweise wiederum über ein Zugband 162. Indem der implantierbare Sensor 112 durch die Engstelle 166 gezogen wird, können die Deckschichten 134, 178 beidseitig auf das Substrat 126, beispielsweise die flexible Leiterplatte, aufgepresst und mit dieser verbunden werden, beispielsweise über die optionalen Klebeschichten 136, 180. Auf diese Weise kann ein implantierbarer Sensor 112 erzeugt werden, dessen äußere Oberfläche vollumfänglich strahlensterilisiert ist, wobei dennoch während der Herstellung eine Strahlenschädigung des mindestens einen elektronischen Bauelements 128 durch den Strahlenschirm 144 verhindert werden kann.

Allgemein können mittels der oben beschriebenen Ausgestaltungen der vorliegenden Erfindung und/oder mit anderen erfindungsgemäßen Ausgestaltungen trotz der notwendigen Strahlensterilisation mit energiereichen Strahlen handelsübliche Elektronikkomponenten als elektronische Bauelemente 128 eingesetzt werden. Die dargestellte Option einer Entnahme des Strahlenschirms 144 aus der Verpackung 114 bringt weitere Vorteile mit sich. So muss beispielsweise der Strahlenschirm 144 nicht mehr an einen Endverbraucher ausgeliefert werden, was eine Kostenersparnis und eine Verkleinerung des Volumens ausgelieferten Produkts bewirken kann. Insbesondere kann das Sensorsystem bei der Anwendung am Patienten ein niedriges Profil aufweisen, da der Strahlenschirm 144 nicht Bestandteil des implantierbaren Sensors 112 ist.

Weiterhin bedingt die in Figur 5 angedeutete Möglichkeit einer Integration weiterer medizinischer Hilfsmittel in die Verpackung 114 zusätzliche Vorteile. So können beispielsweise verschiedene Komponenten, wie der Sensorteil 120, insbesondere die Sensorspitze, die Insertionshilfe 172, insbesondere die Schlitzkanüle, und die optionale, die Haut berührende zweite Klebeschicht 140 gemeinsam in einer als steril dichte Umverpackung ausgestalteten Verpackung 114 sterilisiert werden und nach der Sterilisation ohne Öffnung der Sterilverpackung zusammen montiert werden, beispielsweise indem diese Komponenten innerhalb der Verpackung 114 in eine oder mehrere neue Relativpositionen gebracht werden, insbesondere durch Verformung der Verpackung 114.

### Bezugszeichenliste

- 110: Sterilisationsvorrichtung
- 112: implantierbarer Sensor
- 114: Verpackung
- 116: Innenraum
- 118: Elektronikteil
- 120: Sensorteil
- 122: Sensorelektrode
- 124: Substrat
- 126: Substrat
- 128: elektronisches Bauelement
- 130: erstes Teil
- 132: zweites Teil
- 134: Deckschicht
- 136: erste Klebeschicht
- 138: Abdeckschicht
- 140: zweite Klebeschicht
- 142: Abdeckschicht
- 144: Strahlenschirm
- 146: Bestrahlungsrichtung
- 148: Verbindung
- 150: Finger
- 152: Klemmbereich
- 154: Griff
- 156: erster Verpackungsteil
- 158: Griff
- 160: zweiter Verpackungsteil
- 162: Zugband
- 164: Faltenbalg
- 166: Engstelle
- 168: Wand
- 170: Druckkissen
- 172: Insertionshilfe
- 174: Sollbruchstelle
- 176: Bestrahlungsrichtung

- 178: weitere Deckschicht
- 180: weitere Klebeschicht
- 182: weitere Abdeckschicht
- 184: weitere Verbindung
- 186: weiterer Finger

## Patentansprüche

1. Verfahren zum Sterilisieren eines implantierbaren Sensors (112) zur Erfassung mindestens eines Analyten in einem Körpergewebe, wobei der implantierbare Sensor (112) mindestens einen in das Körpergewebe einbringbaren Sensorteil (120) mit mindestens einer Sensorelektrode (122) zur Erfassung des Analyten und mindestens einen Elektronikteil (118) aufweist, wobei der Elektronikteil (118) mindestens ein elektronisches Bauelement (128) aufweist und mit dem Sensorteil (120) verbunden ist, wobei das Verfahren folgende Schritte aufweist:
a) der implantierbare Sensor (112) wird in mindestens eine Verpackung (114) eingebracht, wobei die Verpackung (114) den implantierbaren Sensor (112) keimdicht abschließt und wobei die Verpackung (114) einen den Elektronikteil (118) abschirmenden Strahlenschirm (144) aufnimmt,
b) der implantierbare Sensor (112) wird in der Verpackung (114) aus mindestens einer Bestrahlungsrichtung (146, 176) mit sterilisierender Strahlung bestrahlt, insbesondere mit Elektronen-Strahlung, wobei der Strahlenschirm (144) das elektronische Bauelement (128) des Elektronikteils (118) gegenüber der sterilisierenden Strahlung abschirmt, wobei der Strahlenschirm (144) derart angeordnet wird, dass der Sensorteil (120) durch die sterilisierenden Strahlung sterilisiert wird.

2. Verfahren nach dem vorhergehenden Anspruch, wobei der implantierbare Sensor (112) nach Durchführung des Verfahrensschritts a) mindestens einen ersten Teil (130) und mindestens einen zweiten Teil (132) aufweist, wobei der erste Teil (130) in Verfahrensschritt b) sterilisiert wird, wobei der zweite Teil (132) in Verfahrensschritt b) durch den Strahlenschirm (144) gegenüber der sterilisierenden Strahlung abgeschirmt wird, wobei nach Durchführung des Verfahrensschritts b) der erste Teil (130) und der zweite Teil (132) innerhalb der Verpackung (114) verbunden werden.

3. Verfahren nach dem vorhergehenden Anspruch, wobei die Verpackung (114) verformbar ausgestaltet ist, wobei bei einer Verformung der Verpackung (114) nach Durchführung des Verfahrensschritts b) der erste Teil (130) und der zweite Teil (132) derart zueinander bewegt werden, dass diese miteinander verbunden werden.

4. Verfahren nach einem der vorhergehenden Ansprüche, wobei der Elektronikteil (118) einen Schichtaufbau aufweist, wobei der Schichtaufbau mindestens eine Deckschicht (134) aufweist, wobei im Verfahrensschritt b) die Deckschicht (134) derart zu dem Strahlenschirm (144) angeordnet wird, dass die Deckschicht (134) zumindest teilweise durch die sterilisierende Strahlung sterilisiert wird, wobei nach Durchführung des Verfahrensschritts b) die Deckschicht (134) innerhalb der Verpackung (114) auf das elektronische Bauelement (128) aufgebracht wird, insbesondere ohne Öffnen der Verpackung (114).

5. Verfahren nach dem vorhergehenden Anspruch, wobei die Verpackung (114) verformbar ausgestaltet ist, wobei bei einer Verformung der Verpackung (114) der implantierbare Sensor (112) durch mindestens eine Engstelle (166) bewegt wird, wobei durch die Engstelle (166) die Deckschicht (134) auf das elektronische Bauelement (128) aufgepresst wird.

6. Verfahren nach dem vorhergehenden Anspruch, wobei bei der Verformung der Verpackung (114) der Strahlenschirm (144) von der Verpackung (114) getrennt wird.

7. Verfahren nach einem der vorhergehenden Ansprüche, wobei im Verfahrensschritt a) zusätzlich mindestens ein medizinisches Hilfsmittel, insbesondere mindestens eine Insertionshilfe (172), in die Verpackung (114) eingebracht wird.

8. Sterilisationsvorrichtung (110) zum Sterilisieren eines implantierbaren Sensors (112) zur Erfassung mindestens eines Analyten in einem Körpergewebe, insbesondere zum Einsatz in einem Verfahren nach einem der vorhergehenden Ansprüche, wobei die Sterilisationsvorrichtung (110) umfasst:
- mindestens einen implantierbaren Sensor (112) zur Erfassung mindestens eines Analyten in einem Körpergewebe, wobei der implantierbare Sensor (112) mindestens einen in das Körpergewebe einbringbaren Sensorteil (120) mit mindestens einer Sensorelektrode (122) zur Erfassung des Analyten und mindestens einen Elektronikteil (118) aufweist, wobei der Elektronikteil (118) mindestens ein elektronisches Bauelement (128) aufweist und mit dem Sensorteil (120) verbunden ist;
- mindestens eine Verpackung (114), wobei die Verpackung (114) den implantierbaren Sensor (112) keimdicht abschließt und wobei die Verpackung (114) mindestens einen das elektronische Bauelement (128) des Elektronikteils (118) während einer Strahlensterilisation abschirmenden Strahlenschirm (144) aufnimmt.

9. Sterilisationsvorrichtung (110) nach dem vorhergehenden Anspruch, wobei der Sensor (112) mindestens einen ersten Teil (130) und mindestens einen zweiten Teil (132) aufweist, wobei der erste Teil (130) bei der Strahlensterilisation in der Verpackung (114) sterilisierbar ist, wobei der zweite Teil (132) bei der Strahlensterilisation durch den Strahlenschirm (144) abgeschirmt wird, wobei nach Durchführung der Strahlensterilisation der erste Teil (130) und der zweite Teil (132) innerhalb der Verpackung (114) verbindbar sind, insbesondere ohne Öffnen der Verpackung (114).

10. Sterilisationsvorrichtung (110) nach dem vorhergehenden Anspruch, wobei die Verpackung (114) verformbar ausgestaltet ist, wobei bei einer Verformung der Verpackung (114) nach Durchführung der Strahlensterilisation der erste Teil (130) und der zweite Teil (132) derart zueinander bewegbar sind, dass diese miteinander verbunden werden.

11. Sterilisationsvorrichtung (110) nach einem der vorhergehenden, eine Sterilisationsvorrichtung (110) betreffenden Ansprüche, wobei der Elektronikteil (118) einen Schichtaufbau aufweist, wobei der Schichtaufbau mindestens eine Deckschicht (134) aufweist, wobei die Verpackung (114) derart ausgestaltet ist, dass während der Strahlensterilisation die Deckschicht (134) derart zu dem Strahlenschirm (144) angeordnet ist, dass die Deckschicht (134) zumindest teilweise durch die sterilisierende Strahlung sterilisierbar ist, während das elektronische Bauelement (128) abgeschirmt ist, wobei die Verpackung (114) derart ausgestaltet ist, dass nach Durchführung der Strahlensterilisation die Deckschicht (134) innerhalb der Verpackung (114) auf das elektronische Bauelement (128) aufgebracht werden kann, insbesondere ohne Öffnen der Verpackung (114).

12. Sterilisationsvorrichtung (110) nach dem vorhergehenden Anspruch, wobei die Verpackung (114) verformbar ausgestaltet ist, wobei bei einer Verformung der Verpackung (114) der implantierbare Sensor (112) durch mindestens eine Engstelle (166) bewegt wird, insbesondere gezogen wird, wobei durch die Engstelle (166) die Deckschicht (134) auf das elektronische Bauelement (128) aufpressbar ist.

13. Sterilisationsvorrichtung (110) nach dem vorhergehenden Anspruch, wobei bei der Verformung der Verpackung (114) der Strahlenschirm (144) von der Verpackung (114) trennbar ist.

14. Sterilisationsvorrichtung (110) nach einem der beiden vorhergehenden Ansprüche, wobei der Strahlenschirm (144) vor dem Verformen der Verpackung (114) von außen in mindestens einen in einen Innenraum (116) der Verpackung (114) hineinragenden Finger (150) der Verpackung (114) eingesteckt ist.

15. Sterilisationsvorrichtung (110) nach einem der drei vorhergehenden Ansprüche, wobei die Verpackung (114) derart verformbar ausgestaltet ist, dass der implantierbare Sensor (112) mit einem ersten Verpackungsteil (156) der Verpackung (114) verbunden ist, wobei der Strahlenschirm (144) mit einem zweiten Verpackungsteil (160) der Verpackung (114) bewegbar ist, wobei während der Verformung der Verpackung (114) der erste Verpackungsteil (156) und der zweite Verpackungsteil (160) derart miteinander verbunden bleiben, dass eine keimdichte Abschirmung des implantierbaren Sensors (112) gewährleistet ist.

## Claims

1. Method for sterilizing an implantable sensor (112) for sensing at least one analyte in a body tissue, wherein the implantable sensor (112) has at'least one sensor part (120) which can be introduced into the body tissue and has at least one sensor electrode (122) for sensing the analyte and at least one electronics part (118), the electronics part (118) having at least one electronic component (128) and being connected to the sensor part (120), the method having the following steps:
a) the implantable sensor (112) is introduced into at least one package (114), the package (114) closing off the implantable sensor (112) such that it is sealed from bacteria and the package (114) accommodating a radiation shield (144) that shields the electronics part (118),
b) the implantable sensor (112) is irradiated in the package (114) with sterilizing radiation from at least one irradiating direction (146, 176), in particular with electron radiation, the radiation shield (144) shielding the electronic component (128) of the electronics part (118) from the sterilizing radiation, the radiation shield (144) being arranged in such a way that the sensor part (120) is sterilized by the sterilizing radiation.

2. Method according to the preceding claim, wherein, after carrying out method step a), the implantable sensor (112) has at least a first part (130) and at least a second part (132), the first part (130) being sterilized in method step b), the second part (132) being shielded from the sterilizing radiation by the radiation shield (144) in method step b), wherein, after carrying out method step b), the first part (130) and the second part (132) are connected within the package (114).

3. Method according to the preceding claim, the package (114) being of a deformable design in such a way that, when there is a deformation of the package (114) after carrying out method step b), the first part (130) and the second part (132) are moved in relation to each other in such a way that they are connected to each other.

4. Method according to one of the preceding claims, the electronics part (118) having a layered structure, the layered structure having at least one overlayer (134), wherein, in method step b), the overlayer (134) is arranged in such a way in relation to the radiation shield (144) that the overlayer (134) is at least partially sterilized by the sterilizing radiation, wherein, after carrying out method step b), the overlayer (134) is applied to the electronic component (128) within the package (114), in particular without opening the package (114).

5. Method according to the preceding claim, the package (114) being of a deformable design in such a way that, when there is a deformation of the package (114), the implantable sensor (112) is moved through at least one constriction (166), the overlayer (134) being pressed onto the electronic component (128) by the constriction (166).

6. Method according to the preceding claim, wherein, during the deformation of the package (114), the radiation shield (144) is separated from the package (114).

7. Method according to one of the preceding claims, wherein, in method step a), at least one medical aid, in particular at least one insertion aid (172), is additionally introduced into the package (114).

8. Sterilizing device (110) for sterilizing an implantable sensor (112) for sensing at least one analyte in a body tissue, in particular for use in a method according to one of the preceding claims, the sterilizing device (110) comprising:
- at least one implantable sensor (112) for sensing at least one analyte in a body tissue, wherein the implantable sensor (112) has at least one sensor part (120) which can be introduced into the body tissue and has at least one sensor electrode (122) for sensing the analyte and at least one electronics part (118), the electronics part (118) having at least one electronic component (128) and being connected to the sensor part (120) ;
- at least one package (114), the package (114) closing off the implantable sensor (112) such that it is sealed from bacteria and the package (114) accommodating at least one radiation shield (144) that shields the electronic component (128) of the electronics part (118) during a radiation sterilization.

9. Sterilizing device (110) according to the preceding claim, the sensor (112) having at least a first part (130) and at least a second part (132), the first part (130) being sterilizable in the package (114) during the radiation sterilization, the second part (132) being shielded by the radiation shield (144) during the radiation sterilization, the first part (130) and the second part (132) being able to be connected within the package (114) after carrying out the radiation sterilization, in particular without opening the package (114).

10. Sterilizing device (110) according to the preceding claim, the package (114) being of a deformable design in such a way that, when there is a deformation of the package (114) after carrying out the radiation sterilization, the first part (130) and the second part (132) can be moved in relation to each other in such a way that they are connected to each other.

11. Sterilizing device (110) according to one of the preceding claims concerning a sterilizing device (110), the electronics part (118) having a layered structure, the layered structure having at least one overlayer (134), wherein the package (114) is designed in such a way that, during the radiation sterilization, the overlayer (134) is arranged in such a way in relation to the radiation shield (144) that the overlayer (134) can be at least partially sterilized by the sterilizing radiation, while the electronic component (128) is shielded, the package (114) being designed in such a way that, after carrying out the radiation sterilization, the overlayer (134) can be applied to the electronic component (128) within the package (114), in particular without opening the package (114).

12. Sterilizing device (110) according to the preceding claim, the package (114) being of a deformable design in such a way that, when there is a deformation of the package (114), the implantable sensor (112) is moved through at least one constriction (166), in particular is pulled, the overlayer (134) being able to be pressed onto the electronic component (128) by the constriction (166).

13. Sterilizing device (110) according to the preceding claim, wherein, during the deformation of the package (114), the radiation shield (144) can be separated from the package (114).

14. Sterilizing device (110) according to one of the two preceding claims, wherein, before the deforming of the package (114), the radiation shield (144) has been inserted from the outside into at least one finger (150) of the package (114) protruding into an interior space (116) of the package (114).

15. Sterilizing device according to one of the three preceding claims, the package (114) being of a deformable design in such a way that the implantable sensor (112) is connected to a first package part (156) of the package (114), the radiation shield (144) being movable with a second package part (160) of the package (114), the first package part (156) and the second package part (160) remaining connected to each other during the deformation of the package (114) in such a way that a bacteria-sealed shielding of the implantable sensor (112) is ensured.

## Revendications

1. Procédé de stérilisation d'un capteur implantable (112) pour la détection d'au moins un analyte dans un tissu corporel, dans lequel le capteur implantable (112) présente au moins un élément capteur (120) pouvant être placé dans le tissu corporel comprenant au moins une électrode de capteur (122) pour la détection de l'analyte et au moins un élément électronique (118), dans lequel l'élément électronique (118) présente au moins un composant électronique (128) et est relié à l'élément capteur (120), dans lequel le procédé présente les étapes suivantes :
a) le capteur implantable (112) est placé dans au moins un emballage (114), l'emballage (114) isolant le capteur implantable (112) contre les germes et l'emballage (114) logeant un écran anti-rayonnement (144) protégeant l'élément électronique (118),
b) le capteur implantable (112) est irradié dans l'emballage (114) à partir d'au moins une direction d'irradiation (146, 176) par un rayonnement stérilisant, en particulier un rayonnement électronique, l'écran anti-rayonnement (144) protégeant le composant électronique (128) de l'élément électronique (118) contre le rayonnement stérilisant, l'écran anti-rayonnement étant disposé de telle sorte que l'élément capteur (120) est stérilisé par le biais du rayonnement stérilisant.

2. Procédé selon la revendication précédente, dans lequel le capteur implantable (112) présente après l'exécution de l'étape de procédé a) au moins un premier élément (130) et au moins un deuxième élément (132), dans lequel le premier élément (130) est stérilisé à l'étape de procédé b), dans lequel le deuxième élément (132) est protégé contre le rayonnement stérilisant à l'étape b) par le biais de l'écran anti-rayonnement (144), dans lequel après l'exécution de l'étape de procédé b) le premier élément (130) et le deuxième élément (132) sont reliés à l'intérieur de l'emballage (114).

3. Procédé selon la revendication précédente, dans lequel l'emballage (114) est configuré pour être déformable, dans lequel lors d'une déformation de l'emballage (114) après l'exécution de l'étape de procédé b) le premier élément (130) et le deuxième élément (132) sont déplacés l'un vers l'autre de telle sorte qu'ils sont reliés l'un à l'autre.

4. Procédé selon l'une des revendications précédentes, dans lequel l'élément électronique (118) présente une structure en couches, dans lequel la structure en couches présente au moins une couche de recouvrement (134), dans lequel à l'étape de procédé b) la couche de recouvrement (134) est disposée par rapport à l'écran anti-rayonnement (144) de telle sorte que la couche de recouvrement (134) est stérilisée au moins partiellement par le biais du rayonnement stérilisant, dans lequel après l'exécution de l'étape de procédé b) la couche de recouvrement (134) est appliquée sur le composant électronique (128) à l'intérieur de l'emballage (114), en particulier sans ouverture de l'emballage (114).

5. Procédé selon la revendication précédente, dans lequel l'emballage (114) est configuré pour être déformable, dans lequel lors d'une déformation de l'emballage (114) le capteur implantable (112) est déplacé à travers au moins un resserrement (166), dans lequel la couche de recouvrement (134) est appuyée sur le composant électronique (128) par le biais du resserrement (166).

6. Procédé selon la revendication précédente, dans lequel lors de la déformation de l'emballage (114) l'écran anti-rayonnement (144) est séparé de l'emballage (114).

7. Procédé selon l'une des revendications précédentes, dans lequel à l'étape de procédé a) en outre au moins un accessoire médical, en particulier au moins un accessoire d'insertion (172), est placé dans l'emballage (114).

8. Dispositif de stérilisation (110) pour la stérilisation d'un capteur implantable (112) pour la détection d'au moins un analyte dans un tissu corporel, en particulier pour une utilisation dans un procédé selon l'une des revendications précédentes, dans lequel le dispositif de stérilisation (110) comprend :
- au moins un capteur implantable (112) pour la détection d'au moins un analyte dans un tissu corporel, le capteur implantable (112) présentant au moins un élément capteur (120) pouvant être placé dans la tissu corporel comprenant au moins une électrode de capteur (122) pour la détection de l'analyte et au moins un élément électronique (118), l'élément électronique (118) présentant au moins un composant électronique (128) et étant relié à l'élément capteur (120) ;
- au moins un emballage (114), l'emballage (114) isolant le capteur implantable (112) contre les germes et l'emballage (114) logeant au moins un écran anti-rayonnement (144) protégeant le composant électronique (128) de l'élément électronique (118) pendant une stérilisation par rayonnement.

9. Dispositif de stérilisation (110) selon la revendication précédente, dans lequel le capteur (112) présente au moins un premier élément (130) et au moins un deuxième élément (132), dans lequel le premier élément (130) est stérilisable lors de la stérilisation par rayonnement dans l'emballage (114), dans lequel le deuxième élément (132) est protégé lors de la stérilisation par rayonnement par le biais de l'écran anti-rayonnement (144), dans lequel après l'exécution de la stérilisation par rayonnement le premier élément (130) et le deuxième élément (132) peuvent être reliés à l'intérieur de l'emballage (114), en particulier sans ouverture de l'emballage (114).

10. Dispositif de stérilisation (110) selon la revendication précédente, dans lequel l'emballage (114) est configuré pour être déformable, dans lequel lors de la déformation de l'emballage (114) après l'exécution de la stérilisation par rayonnement le premier élément (130) et le deuxième élément (132) sont déplaçables l'un vers l'autre de telle sorte qu'ils peuvent être reliés l'un à l'autre.

11. Dispositif de stérilisation (110) selon l'une des revendications précédentes concernant un dispositif de stérilisation (110), dans lequel l'élément électronique (118) présente une structure en couches, dans lequel la structure en couches présente au moins une couche de recouvrement (134), dans lequel l'emballage (114) est configuré de telle sorte que pendant la stérilisation par rayonnement la couche de recouvrement (134) est disposée par rapport à l'écran anti-rayonnement (144) de telle sorte que la couche de recouvrement (134) est stérilisable au moins partiellement par le biais du rayonnement stérilisant, tandis que le composant électronique (128) est protégé, dans lequel l'emballage (114) est configuré de telle sorte qu'après l'exécution de la stérilisation par rayonnement la couche de recouvrement (134) peut être appliquée sur le composant électronique (128) à l'intérieur de l'emballage (114), en particulier sans ouverture de l'emballage (114).

12. Dispositif de stérilisation (110) selon la revendication précédente, dans lequel l'emballage (114) est configuré pour être déformable, dans lequel lors d'une déformation de l'emballage (114) le capteur implantable (112) est déplacé à travers au moins un resserrement (166), en particulier tiré, dans lequel la couche de recouvrement (134) peut être appuyée sur le composant électronique (128) par le biais du resserrement (166).

13. Dispositif de stérilisation (110) selon la revendication précédente, dans lequel lors de la déformation de l'emballage (114) l'écran anti-rayonnement (144) est séparable de l'emballage (114).

14. Dispositif de stérilisation (110) selon l'une des deux revendications précédentes, dans lequel avant la déformation de l'emballage (114) l'écran anti-rayonnement (144) est introduit depuis l'extérieur dans au moins un doigt (150) de l'emballage (114) saillant dans un espace interne (116) de l'emballage (114).

15. Dispositif de stérilisation (110) selon l'une des trois revendications précédentes, dans lequel l'emballage (114) est configuré pour être déformable de telle sorte que le capteur implantable (112) est relié à un premier élément d'emballage (156) de l'emballage (114), dans lequel l'écran anti-rayonnement (144) est déplaçable avec un deuxième élément d'emballage (160) de l'emballage (114), dans lequel pendant la déformation de l'emballage (114) le premier élément d'emballage (156) et le deuxième élément d'emballage (160) restent reliés l'un à l'autre de telle sorte qu'une protection du capteur implantable (112) contre les germes est assurée.
